(19) Europäisches Patentamt
European Patent Office
Office européen des brevets

(11) EP 1 967 203 A1

(12) **EUROPEAN PATENT APPLICATION**

(43) Date of publication:
**10.09.2008 Bulletin 2008/37**

(21) Application number: **08101785.7**

(22) Date of filing: **15.07.1999**

(51) Int Cl.:
***A61K 39/085*** *(2006.01)* ***C07H 5/06*** *(2006.01)*
***C07H 11/00*** *(2006.01)* ***C07H 13/12*** *(2006.01)*
***C08B 37/00*** *(2006.01)*

(84) Designated Contracting States:
**AT BE CH CY DE DK ES FI FR GB GR IE IT LI LU MC NL PT SE**

(30) Priority: **15.07.1998 US 93117 P**

(62) Document number(s) of the earlier application(s) in accordance with Art. 76 EPC:
**99934091.2 / 1 096 952**

(71) Applicant: **The Brigham and Women's Hospital Boston MA 02115-5817 (US)**

(72) Inventors:
- **Pier, Gerald B.**
  **Brookline, MA 02146 (US)**
- **McKenny, David**
  **Quincy, MA 02170 (US)**
- **Wang, Ying**
  **Channing Laboratory, Department of Medicine**
  **Boston, MA 02115 (US)**

(74) Representative: **Furlong, Isla Jane et al**
**Venner Shipley LLP**
**20 Little Britain**
**London**
**EC1A 7DH (GB)**

Remarks:
This application was filed on 20-02-2008 as a divisional application to the application mentioned under INID code 62.

(54) **Polysaccharide vaccine for staphylococcal infections**

(57) The invention relates to compositions of the capsular polysaccharide/adhesin (PS/A) of staphylococci. The PS/A may be isolated or synthesized and includes various modifications to the structure of native PS/A based on the chemical characterization of PS/A. The invention also relates to the use of the PS/A as a vaccine for inducing active immunity to infections caused by *Staphylococcus aureus, S. epidermidis,* other related coagulase-negative staphylococci and organisms carrying the *ica* (intracellular adhesin) locus, and to the use of antibodies directed to PS/A for inducing passive immunity to the same class of infections.

EP 1 967 203 A1

## Description

### Field Of The Invention

The present invention relates to polysaccharide compositions useful for inducing immunity for the prevention of *staphylococcal* infections. The invention also relates to methods of making and using diagnostic kits and for inducing active and passive immunity using the polysaccharide material and antibodies thereto. In particular, the polysaccharide is a capsular polysaccharide/adhesin.

### Background Of The Invention

*Staphylococci* are gram-positive bacteria which normally inhabit and colonize the skin and mucus membranes of humans. If the skin or mucus membrane becomes damaged during surgery or other trauma, the *staphylococci* may gain access to internal tissues causing infection to develop. If the *staphylococci* proliferate locally or enter the lymphatic or blood system, serious infectious complications such as those associated with *staphylococcal bacteremia* may result. Complications associated with *staphylococcal bacteremia* include septic shock, endocarditis, arthritis, osteomyelitis, pneumonia, and abscesses in various organs.

*Staphylococci* include both coagulase positive organisms that produce a free coagulase and coagulase negative organisms that do not produce this free coagulase. *Staphylococcus aureus* is the most common coagulase-positive form of *staphylococci. S. aureus* generally causes infection at a local site, either extravascular or intravascular, which ultimately may result in *bacteremia. S. aureus* is also a leading cause of acute osteomyelitis and causes a small number of *staphylococcal* pneumonia infections. Additionally, *S. aureus* is responsible for approximately 1-9% of the cases of bacterial meningitis and 10-15% of brain abscesses. There are at least twenty-one known species of coagulase-negative *staphylococci,* including *S. epidermidis, S. saprophyticus, S hominis, S. warneri, S. haemolyticus, S. saprophiticus, S. cohnii, S. xylosus, S. simulans,* and S. *capitis. S. epidermidis* is the most frequent infection-causing agent associated with intravenous access devices and the most frequent isolate in primary nosocomial *bacteremias. S. epidermidis* is also associated with prosthetic valve endocarditis.

*Staphylococcus* is also a common source of bacterial infection in animals. For instance, *staphylococcal* mastitis is a common problem in ruminants including cattle, sheep, and goats. The disease is generally treated with antibiotics to reduce the infection but the treatment is a costly procedure and still results in a loss of milk production. The most effective vaccines identified to date are live, intact *S. aureus* vaccines administered subcutaneously. The administration of live vaccines, however, is associated with the risk of infection. For that reason, many researchers have attempted to produce killed S. *aureus* vaccines and/or to isolate capsular polysaccharides or cell wall components which will induce immunity to *S. aureus.* None of these attempts, however, has been successful.

*S. aureus* includes a cell wall component composed of a peptidoglycan complex which enables the organism to survive under unfavorable osmotic conditions and also includes a unique teichoic acid linked to the peptidoglycan. External to the cell wall a thin polysaccharide capsule coats most isolates of *S. aureus.* This serologically distinct capsule can be used to serotype various isolates of *S. aureus.* Many of the clinically significant isolates have been shown to include two capsular types, CP5 and CP8.

Type CP5 has the following chemical structure:

→4)-β-D-Man*p*NAcA3Ac-(1→4)-α-L-Fuc*p*NAc-(1→3)-β-D-Fuc*p*NAc-(1→

Type CP8 has the following chemical structure:

→3)-(3-ß-D-Man*p*NAcA4Ac-(1→3)-α-L-Fuc*p*NAc-(1→3)-β-D-Fuc*p*NAc-(1→

Studies on over 1600 *S. aureus* isolates showed that 93% were of either type 5 or type 8 capsular polysaccharides. Additionally, more than 80% of the *S. aureus* isolated from sheep, goats, and cows with mastitis and chickens with osteomyelitis have been demonstrated to include at least one of these two capsular types. Although CP5 and CP8 are structurally similar, they have been demonstrated to be immunologically distinct

### Summary Of The Invention

The present invention relates to methods and products for passive and active immunization of humans and animals against infection by coagulase-negative and coagulase-positive *staphylococci.* It has been discovered, according to the invention, that a true "capsule" of *S. aureus* extends out beyond the CP5 or CP8 layer or is produced instead of

the CP5 or CP8 layer. This material, which is the capsular polysaccharide/adhesin (PS/A) antigen, has been purified to near homogeneity and the structure identified. The native PS/A antigen which can be isolated from *Staphylococci* such as *S. aureus* and *S. epidermis* is a high molecular weight polyglucosamine which is heavily substituted with succinic acid residues, and is referred to as poly-beta-1-6-D-N-succinylglucosamine (PNSG).
The isolated and purified PS/A antigen has been found to be highly immunogenic *in vivo.*

In one aspect the invention is a composition of a pure PS/A having the following structure:

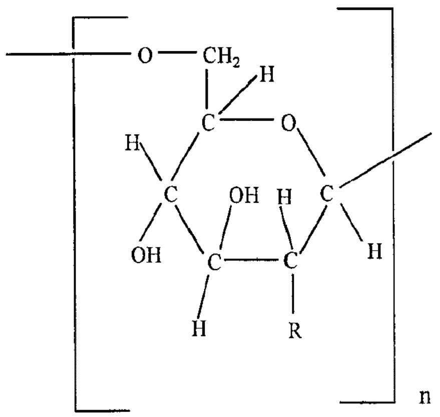

wherein n is an integer greater than or equal to 3, wherein R is selected from the group consisting of $-NH-CO-(CH_2)_m-COOH$, $-NH-(CH_2)_m-(COOH)_2$, $-NH-(CH_2)_m-COOH$, and $-NH_2$ wherein m is 2-5, and wherein at least 10% of the R groups are selected from the group consisting of $-NH-CO-(CH_2)_m-COOH$, $-NH-(CH_2)_m-(COOH)_2$, and $-NH-(CH_2)_m-COOH$. In other preferred embodiments at least 50%, 75%, or 90% of the R groups are selected from the group consisting of $-NH-CO-(CH_2)_m-COOH$, $-NH-(CH_2)_m-(COOH)_2$, and $-NH-(CH_2)_m-COOH$. The composition in one embodiment is formulated as a vaccine. In preferred embodiments, the PS/A is at least 95%, 97% or 99% pure. The preferred backbone linkage is a β 1-6 linkage.

In a preferred embodiment the monomeric unit of PS/A is substituted with succinate. In this embodiment the R group is $-NH-CO-CH_2-CH_2-COOH$. In other embodiments the R group is selected from the group consisting of $-NH-CO-CH_2-CH_2-CH_2-COOH$, $-NH-CO-CH_2-CH_2-CH_2-CH_2-COOH$, $-NH-CO-CH_2-CH_2-CH_2-CH_2-CH_2-COOH$, $NH-CH_2-CH_2-COOH$, $-NH-CH_2-CH_2-CH_2-COOH$, $-NH-CH_2-CH_2-CH_2-CH_2-COOH$, $-NH-CH_2-CH_2-CH_2-CH_2-CH_2-COOH$, $-NH-CH_2-CH_2-(COOH)_2$, $-NH-CH_2-CH_2-CH_2-(COOH)_2$, $-NH-CH_2-CH_2-CH_2-CH_2-(COOH)_2$, and $-NH-CH_2-CH_2-CH_2-CH_2-CH_2-(COOH)_2$. The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

The PS/A of the invention is a polymer of 3 or more repeating monomeric units and therefore may have a low molecular weight. In a preferred embodiment of the invention the PS/A has a molecular weight of greater than 25,000. In another preferred embodiment the PS/A has a molecular weight of greater than 30,000. In yet another embodiment the PS/A has a molecular weight of greater than 100,000.

The composition may also include a carrier compound conjugated to the PS/A. When the PS/A is used as a vaccine and the PS/A has a low molecular weight it is preferred that the PS/A be conjugated to a carrier compound. In one embodiment the carrier compound is conjugated to the PS/A through a linker.

In another embodiment the PS/A is substantially free of phosphate. In yet another embodiment the PS/A is substantially free of teichoic acid.

In another aspect the invention is a composition of a PS/A having the following structure:

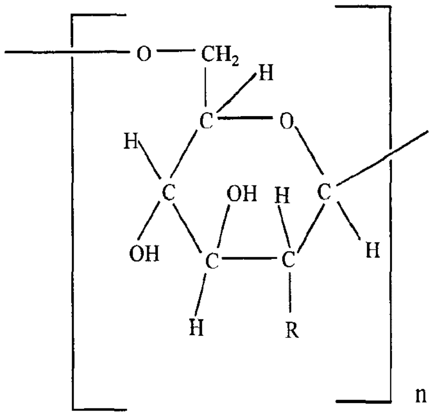

wherein n is an integer greater than or equal to 3, wherein R is selected from the group consisting of -NH-CO-$(CH_2)_{3-5}$-COOH, -NH-$(CH_2)_m$-$(COOH)_2$, -NH-$(CH_2)_m$-COOH, and -$NH_2$, wherein m is 2-5, and wherein at least 10% of the R groups are selected from the group consisting of -NH-CO-$(CH_2)_{3-5}$-COOH, -NH-$(CH_2)_m$-$(COOH)_2$, and -NH-$(CH_2)_m$-COOH. In other preferred embodiments at least 50%, 75%, or 90% of the R groups are selected from the group consisting of --NH-CO-$(CH_2)_{3-5}$-COOH, -NH-$(CH_2)_m$-$(COOH)_2$, and -NH-$(CH_2)_m$-COOH. The composition in one embodiment is formulated as a vaccine. The preferred backbone linkage is a β 1-6 linkage.

In one embodiment the PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

In a preferred embodiment of the invention the PS/A has a molecular weight of greater than 25,000. In another preferred embodiment the PS/A has a molecular weight of greater than 30,000. In yet another embodiment the PS/A has a molecular weight of greater than 100,000.

The composition may also include a carrier compound conjugated to the PS/A. When the PS/A is used as a vaccine and the PS/A has a low molecular weight it is preferred that the PS/A be conjugated to a carrier compound. In one embodiment the carrier compound is conjugated to the PS/A through a linker.

According to another aspect the invention is a composition of a PS/A having the following structure:

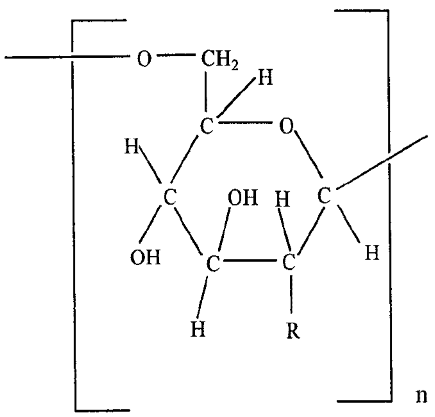

wherein n is an integer greater than or equal to 3, wherein R is selected from the group consisting of -NH-CO-$CH_2$-$CH_2$-COOH and -$NH_2$ and wherein between 10 and 90% of the R groups are -NH-CO-$CH_2$-$CH_2$-COOH. In other preferred embodiments between 20 and 80%, 30 and 70%, or 40 and 60% of the R groups are -NH-CO-$CH_2$-$CH_2$-COOH. The composition in one embodiment is formulated as a vaccine. The preferred backbone linkage is a β 1-6 linkage.

In one embodiment the PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

In a preferred embodiment of the invention the PS/A has a molecular weight of greater than 25,000. In another preferred embodiment the PS/A has a molecular weight of greater than 30,000. In yet another embodiment the PS/A has a molecular weight of greater than 100,000.

The composition may also include a carrier compound conjugated to the PS/A. When the PS/A is used as a vaccine and the PS/A has a low molecular weight it is preferred that the PS/A be conjugated to a carrier compound. In one embodiment the carrier compound is conjugated to the PS/A through a linker.

According to yet another aspect of the invention a composition of a PS/A composed of repeating monomer units wherein the monomer unit is selected from the group consisting of β-1-6 polyglucose, α-1-4 polyglucose, α-1-3 polyglucose, β-1-4 polyglucose, β-1-3 polyglucose, and a α-1-4 polyglactosamine and wherein C2 of the monomer unit is substituted with an R group selected from the group consisting of $-NH-CO-(CH_2)_m-COOH$, $-NH-(CH_2)_m-(COOH)_2$, $-NH-(CH_2)_m-COOH$, and $-NH_2$, wherein m is 2-5, and wherein at least 10% of the R groups are selected from the group consisting of $-NH-CO-(CH_2)_m-COOH$, $-NH-(CH_2)_m-(COOH)_2$, and $-NH-(CH_2)_m-COOH$ is provided. The composition in one embodiment is formulated as a vaccine.

Preferably the monomer unit is selected from the group consisting of β-1-6 polyglucose, α-1-4 polyglucose, α-1-3 polyglucose, β-1-4 polyglucose, and β-1-3 polyglucose. Preferably the polyglucose backbone is in an α-1-6 formation. In another embodiment the polyglucose backbone is in a β-1-4 formation. According to another embodiment the polyglucose backbone is in a β-1-3 formation.

According to a preferred embodiment at least 50%, 75%, or 90% of the R groups are selected from the group consisting of $-NH-CO-(CH_2)_m-COOH$, $-NH-(CH_2)_m-(COOH)_2$, and $-NH-(CH_2)_m-COOH$.

In a preferred embodiment the monomeric unit of PS/A is substituted with succinate. In this embodiment the R group is $-NH-CO-CH_2-CH_2-COOH$. In other embodiments the R group is selected from the group consisting of $-NH-CO-CH_2-CH_2-CH_2-COOH$, $-NH-CO-CH_2-CH_2-CH_2-CH_2-COOH$, $-NH-CO-CH_2-CH_2-CH_2-CH_2-CH_2-COOH$, $-NH-CH_2-CH_2-COOH$, $-NH-CH_2-CH_2-CH_2-COOH$, $-NH-CH_2-CH_2-CH_2-CH_2-COOH$, $-NH-CH_2-CH_2-CH_2-CH_2-CH_2-COOH$, $-NH-CH_2-CH_2-(COOH)_2$, $-NH-CH_2-CH_2-CH_2-(COOH)_2$, $-NH-CH_2-CH_2-CH_2-CH_2-(COOH)_2$, and $-NH-CH_2-CH_2-CH_2-CH_2-CH_2-(COOH)_2$. The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

The PS/A of the invention is a polymer of 3 or more repeating monomeric units and therefore may have a low molecular weight. In a preferred embodiment of the invention the PS/A has a molecular weight of greater than 25,000. In another preferred embodiment the PS/A has a molecular weight of greater than 30,000. In yet another embodiment the PS/A has a molecular weight of greater than 100,000.

The composition may also include a carrier compound conjugated to the PS/A. When the PS/A is used as a vaccine and the PS/A has a low molecular weight it is preferred that the PS/A be conjugated to a carrier compound. In one embodiment the carrier compound is conjugated to the PS/A through a linker.

In another embodiment the PS/A is substantially free of phosphate. In yet another embodiment the PS/A is substantially free of teichoic acid.

According to another aspect of the invention a composition of a PS/A having the following structure:

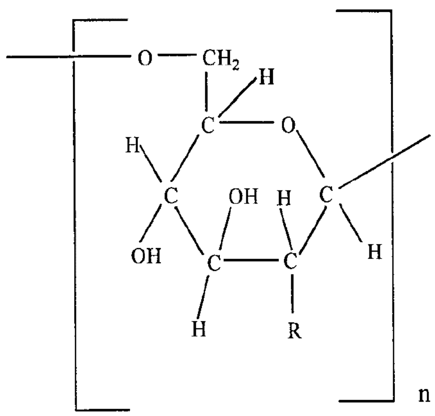

wherein n is an integer greater than or equal to 3, wherein R is selected from the group consisting of $-NH-CO-(CH_2)_m-COOH$, $-NH-(CH_2)_m-(COOH)_2$, $-NH-(CH_2)_m-COOH$, and $-NH_2$, wherein m is 2-5, and wherein at least 10% of the R groups are selected from the group consisting of $-NH-CO-(CH_2)_m-COOH$, $-NH-(CH_2)_m-(COOH)_2$, and $-NH-(CH_2)_m-COOH$ and wherein the PS/A is substantially free of teichoic acid is provided. In other preferred embodiments

at least 50%, 75%, or 90% of the R groups are selected from the group consisting of $-NH-CO-(CH_2)_m-COOH$, $-NH-(CH_2)_m-(COOH)_2$, and $-NH-(CH_2)_m-COOH$. The composition in one embodiment is formulated as a vaccine. The preferred backbone linkage is a β 1-6 linkage.

In a preferred embodiment the monomeric unit of PS/A is substituted with succinate. In this embodiment the R group is $-NH-CO-CH_2-CH_2-COOH$. In other embodiments the R group is selected from the group consisting of $-NH-CO-CH_2-CH_2-CH_2-COOH$, $-NH-CO-CH_2-CH_2-CH_2-CH_2-COOH$, $-NH-CO-CH_2CH_2-CH_2CH_2-CH_2-COOH$, $-NH-CH_2-CH_2-COOH$, $-NH-CH_2-CH_2-CH_2-COOH$, $-NH-CH_2$ $CH_2-CH_2-CH_2-COOH$, $-NH-CH_2-CH_2-CH_2-CH_2-CH_2-COOH$, $-NH-CH_2-CH_2-(COOH)_2$, $-NH-CH_2-CH_2-CH_2-(COOH)_2$, , $-NH-CH_2-CH_2-CH_2-CH_2-(COOH)_2$, and $-NH-CH_2-CH_2-CH_2-CH_2-CH_2-(COOH)_2$. The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

The PS/A of the invention is a polymer of 3 or more repeating monomeric units and therefore may have a low molecular weight. In a preferred embodiment of the invention the PS/A has a molecular weight of greater than 25,000. In another preferred embodiment the PS/A has a molecular weight of greater than 30,000. In yet another embodiment the PS/A has a molecular weight of greater than 100,000.

The composition may also include a carrier compound conjugated to the PS/A. When the PS/A is used as a vaccine and the PS/A has a low molecular weight it is preferred that the PS/A be conjugated to a carrier compound. In one embodiment the carrier compound is conjugated to the PS/A through a linker.

In another embodiment the PS/A is substantially free of phosphate.

In another aspect the invention is a polysaccharide of a pure PS/A prepared according to the following method: extracting a crude PS/A preparation from a bacterial culture; isolating a high-molecular weight PS/A-enriched material from the crude PS/A preparation; precipitating an impure PS/A from the high-molecular weight PS/A-enriched material; incubating the impure PS/A with a base to produce a semi-pure PS/A preparation; neutralizing the preparation with an acid; and incubating the neutralized preparation in hydrofluoric acid to produce the pure impure PS/A.

In a preferred embodiment the bacterial culture is a *Staphylococcus aureus* culture. In another preferred embodiment the bacterial culture is a coagulase negative *Staphylococci* culture.

In another aspect the invention is a method of inducing active immunity to infection by *staphylococci* in a subject. The method includes the step of administering to a subject an effective amount for inducing active immunity to *staphylococci* of any of the above-described compositions.

In one embodiment the method is a method for inducing immunity to infection by *staphylococcus aureus.* In another embodiment the method is a method for inducing immunity to infection by *staphylococcus epidermis.*

According to another aspect of the invention a method of inducing passive immunity to infection by *staphylococcus aureus* in a subject is provided. The method includes the step of administering to a subject an effective amount for inducing opsonization of *staphylococcus aureus* of an anti PS/A antibody.

*Staphylococcus aureus,* is the most common cause of bacterial infection in hospitalized patients. According to an aspect of the invention a method of inducing active immunity to infection by *staphylococcus aureus* in a subject is provided. The method includes the step of administering to a subject an effective amount for inducing active immunity to *staphylococcus aureus* of a high molecular weight polysaccharide having a polyglucosamine backbone wherein at least 10% of individual glucosamine units of the polyglucosamine backbone are conjugated to succinate. In a preferred embodiment the polyglucosamine backbone is in a β-1-6 formation.

A method of preparing a pure polysaccharide is another aspect of the invention. The method includes the steps of extracting a crude PS/A preparation from a bacterial culture, isolating a high-molecular weight PS/A-enriched material from the crude PS/A preparation, precipitating an impure PS/A from the high-molecular weight PS/A-enriched material, incubating the impure PS/A with a base or acid to produce a semi-pure PS/A preparation, neutralizing the preparation, and treating the neutralized preparation to produce the pure PS/A.

In some embodiments the impure PS/A is incubated with a base, neutralized with an acid, and then treated with hydrofluoric acid to produce a pure PS/A. In other embodiments the impure PS/A is incubated with a base or an acid and neutralized with an acid or base, respectively. This preparation is then treated by dialysis against deionized water, lyophilized and resuspended in a buffer.

In another aspect of the invention another method for preparing a pure polysaccharide is provided. The method includes the step of preparing an acid or base solution by incubating a bacterial culture with a strong base or a strong acid. The acid or base solution is then neutralized to pH 7 to produce a crude antigen suspension. The crude antigen suspension is dialyzed against a solution such as deionized water and insoluble crude antigen can be collected. In some embodiments the insoluble crude antigen can be lyophilized and then resuspended in a buffer. In some embodiments the buffer is selected from the group consisting of 50 mM PBS and 100 mM Tris with 150 mM NaCl. In other embodiments

the strong base or acid is greater than 1 M NaOH or HCL. In other embodiments the strong base or acid is 5 M NaOH or HCL. In another embodiment the bacterial culture extract is stirred in the strong base or acid for 18-24 hours. In yet another embodiment the strong base or acid extraction is repeated.

In another aspect the invention is a composition of an anti-PS/A antibody, wherein the antibody induces opsonization of *staphylococcus aureus* and wherein the antibody does not cross-react with an impure PS/A preparation. In a preferred embodiment the anti-PS/A antibody is a monoclonal antibody. In another preferred embodiment the anti-PS/A antibody is a polyclonal antibody.

Each of the limitations of the invention can encompass various embodiments of the invention. It is, therefore, anticipated that each of the limitations of the invention involving any one element or combinations of elements can be included in each aspect of the invention.

## Brief Description Of The Drawings

Figure 1 is an NMR spectra of PS/A from *S. aureus* strain MN8.

Figure 2 is a graph depicting the results of an ELISA inhibition showing the ability of various strains of *Staphylococcus aureus* grown overnight two times in brain heart infusion broth supplemented with 0.5% glucose to remove antibodies binding to PS/A antigen that has sensitized the ELISA plate (speckled bars). As a control fro specificity a separate aliquot of the same bacteria was treated with 0.4 M sodium metaperiodate to destroy the PS/A antigen on the surface of the *S. aureus* strains (grey bars). The percentage inhibition of binding was determined from the ratio of the optical density achieved using antibody to PS/A adsorbed with the test strain and antibody to PS/A adsorbed with a known PS/ A negative strain (S. *epidermidis* sn-3).

Figure 3 is a graph depicting the ability of a PS/A antigen to induce active immunity in mice against *S. aureus* infection.

Figure 4 is a graph depicting the ability of an anti-PS/A antibody to induce passive immunity in mice against *S. aureus* infection.

Figure 5 shows a photograph of a PCR fragment gel.

Figure 6 shows a photograph of a Southern blot of *ica* (intercellular adhesin locus) genes in *S. aureus.* PCR was performed on chromosomal DNA from controls and eight isolates of *S. Aureus:* (1) molecular weight markers (2) *S. Carnosus* TM300, negative control (3) positive control DNA from S. *Epidermis* RP62A *staphylococcus aureus* strains (4) Reynolds (5) MN8 (6) 5827 (7) S836 (8) Vas (9) VP (10) 265 (11) Por).

## Brief Description Of The Sequences

SEQ. ID. NO. 1 is nucleic acid sequence of the *ica* locus which has been deposited in GenBank under accession number U43366. (Prior Art)

SEQ. ID. NO. 2 is a forward primer sequence for *ica.*

SEQ. ID. NO. 3 is a backward primer sequence for *ica.*

## Detailed Description Of The Invention

The invention relates to a microcapsule from *Staphylococcal* bacteria which is useful for inducing immunity to bacterial infection and also for producing antibodies for diagnostic and therapeutic purposes. The microcapsule is a PS/A preparation which can be isolated from *Staphylococcus aureus* or coagulase-negative *staphylococci,* or can be synthesized *de novo.* PS/A has not previously been identified as being a component of the *Staphylococcus aureus* extracellular layer. Prior to the instant invention, it was demonstrated that two chemically related serologic types of capsule, termed capsular polysaccharide CP5 and CP8 were produced by 90% of *Staphylococcus aureus* strains. It was believed that these capsular polysaccharides were responsible for inducing immune recognition of *S. aureus.* It has been discovered according to the invention that *S. aureus* includes a true capsule which either extends out beyond or replaces the CP5 or CP8 layer and is responsible for provoking an immune response. As demonstrated in the examples presented herein, the PS/A antigen can produce opsonic protective antibodies against *S. aureus* and coagulase-negative *staphylococci* when administered *in vivo.*

Prior to the invention, the inventor of the instant application identified and characterized a portion of the capsule of coagulase-negative *staphylococci,* which was PS/A (U.S. Patent No. 5,055,455, issued to Gerald B. Pier). It was found that the PS/A of coagulase negative *staphylococcus* is a component of the cell surface and biofilm layer and is involved in protecting the bacterial cell from host defenses, such as opsonophagocytosis (Kojima, Y., M. Tojo, D. A. Goldmann, T. D. Tosteson and G. B. Pier. (1990) J Infect Dis.162:435. Tojo, M., N. Yamashita, D. A. Goldmann and G. B. Pier. (1988) J Infect Dis. 157:713. Goldmann, D. A. and G. B. Pier. (1993) Clin Microbiol Rev. 6:176.) The chemical

structure of the PS/A, however, was not identified because of the difficulty associated with purifying the isolated PS/A. It was only possible to achieve a preparation of approximately 90% purity. According to the methods of the instant invention, the PS/A antigen has been isolated and purified to achieve a pure PS/A preparation.

As used herein, a "pure capsular polysaccharide/adhesin" is a PS/A preparation which has been isolated or synthesized and which is greater than 92% free of contaminants. Preferably, the material is greater than 95% or even greater than 97% or 99% free of contaminants. The degree of purity of the PS/A antigen can be assessed by any means known in the art. For example, the purity can be assessed by chemical analysis assays as well as gas chromatography and nuclear magnetic resonance to verify structural aspects of the material.

One major contaminant of the prior art PS/A antigen was phosphate containing teichoic acid. The teichoic acid contamination of the prior art antigen interfered with both the chemical characterization and the immunogenicity of the antigen. The procedures used in the prior art to isolate PS/A were not sufficient to remove contaminating teichoic acid from the PS/A preparation, making the chemical characterization of the PS/A antigen impossible. The methods of the invention described herein are capable of producing an isolated PS/A antigen preparation which is substantially free of teichoic acid. A PS/A preparation that is substantially free of teichoic acid is one which has less than 1.0% phosphate. In some embodiments the PS/A preparation that is substantially free of teichoic acid is one which has less than 0.1 % phosphate. The amount of phosphate present in the sample can be assessed by any means known in the art. As described in the examples below the amount of phosphate contamination can be assessed using the methods described in Keleti, G. and W.H. Lederer, (1974) Handbook of Micromethods for the Biological Sciences Van Nostrand Reinhold Co., New York, which is hereby incorporated by reference. Briefly, the assay is performed as follows: to 100 μg of sample 100 μl of a solution made by adding together 43.5 ml of water, 6.5 ml of 70% perchloric acid ($HClO_4$) and 50 ml of 20 N sulfuric acid ($H_2SO_4$) is added. This is heated at 95 °C for 2 hours in a tube with a marble on top of it. The mixture is next placed in an oven at 165 °C and heated for an additional 2 hours then cooled to room temperature. Next, one ml of reagent 5, made by the following method, is added to the sample:

Reagent 1:1.36 grams of sodium acetate .$3H_20$ dissolved in 10 ml water.
Reagent 2: 500 mg ammonium molybdate dissolved in 20 ml water.
Reagent 3: 2 ml of reagent 1, 2 ml of reagent 2 and 16 ml of water.
Reagent 4: 2 gm ascorbic acid dissolved in 20 ml water, prepared immediately prior to use.
Reagent 5: Add in an ice bath 9 ml of reagent 3 and 1 ml of reagent 4.

After adding reagent 5 the tubes are mixed throughly and the optical density read at 820 nanometers in a spectrophotometer. A standard curve consisting of sodium phosphate monobasic (range of 0.1-5 μg per tube) is used to calculate the amount of phosphate present in the test samples. (Lowry, O.H., N.R. Roberts, K.Y. Leiner, M.L. Wu and A. L. Farr., (1954), Biol. Chem. 207, 1.)

In one aspect, the invention is a composition of a pure PS/A having the following structure:

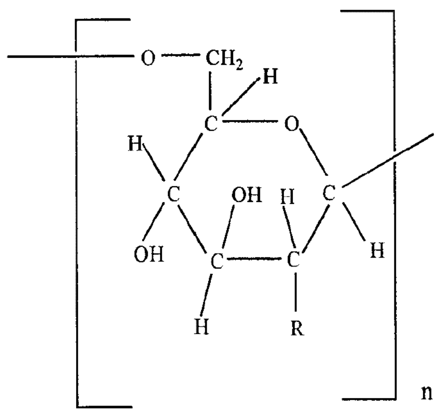

wherein n is an integer greater than or equal to 3, wherein R is selected from the group consisting of -NH-CO-$(CH_2)_m$-COOH, -NH-$(CH_2)_m$-$(COOH)_2$, -NH-$(CH_2)_m$-COOH, , and -$NH_2$
wherein m is 2-5, and wherein at least 10% of the R groups are selected from the group consisting of -NH-CO-$(CH_2)_m$-COOH, -NH-$(CH_2)_m$-$(COOH)_2$, and -NH-$(CH_2)_m$-COOH. The native material has a β 1-6 linkage. This is the

preferred backbone linkage in the PS/A antigen of the invention.

In this aspect of the invention the PS/A antigen is a homopolymer of at least partially substituted glucosamine residues linked to one another. At least 10% of the glucosamine residues are substituted with a short chain fatty acid group of the general formula $COOH-(CH_2)_m-COOH$, $CH_3-(CH_2)_{m-1}-(COOH)_2$, or $CH_3-(CH2)_{m-1}-COOH$ wherein m = 2-5. In other embodiments, the glucosamine residues can be substituted with 20%, 30%, 40%, 50%, 60%, 70%, 80%, 90%, 95%, 97%, or 100% of a short chain fatty acid of the formulas provided above. Preferably, the glucosamine residues are substituted with a dicarboxcylic acid of the formula $NH-CO-(CH_2)_m-COOH$. More preferably, the glucosamine residues are substituted with a succinic acid. The substitution may be a homo-or hetero-substitution. A homo-substitution is one in which the glucosamine residues are substituted with a single type of substituent. For instance, the glucosamine residues may be substituted only with succinic acid residues. In the hetero-substitution, the glucosamine residues may be substituted with more than one type of substituent. For instance, some of the glucosamine residues may be substituted with succinic acid and others may be substituted with butyric acid. A composition of PS/A according to the invention includes all homo-substituted PS/A molecules or all hetero-substituted PS/A molecules or a combination thereof.

The PS/A preferably is a polymer composed of monomers selected from the following chemical structures:

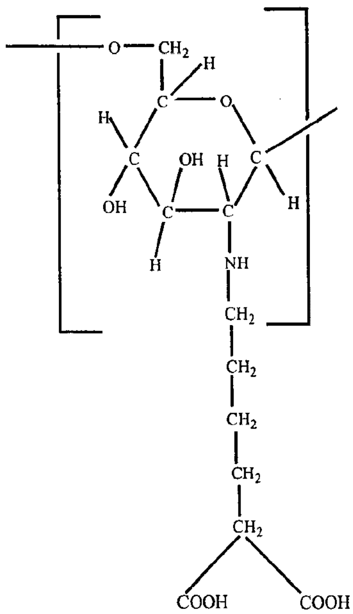
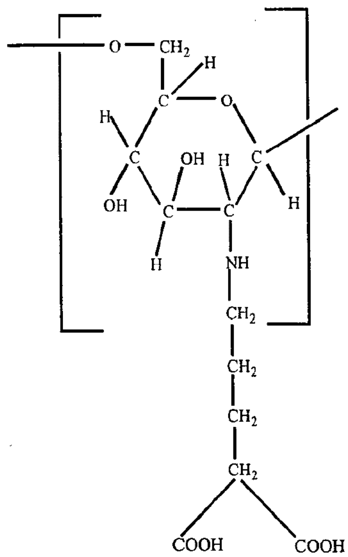
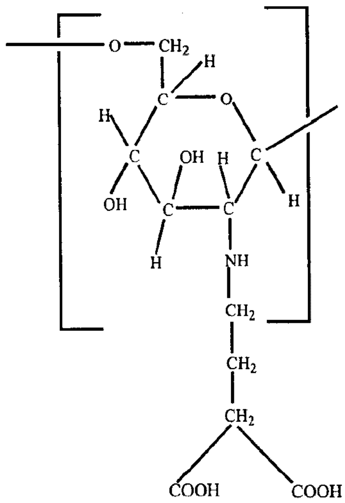
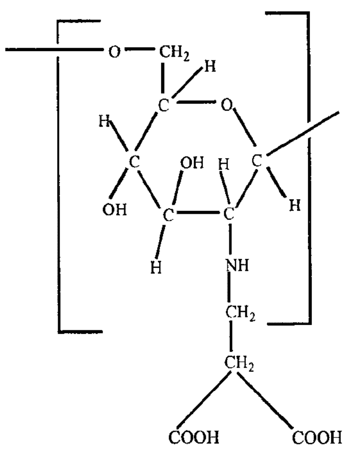
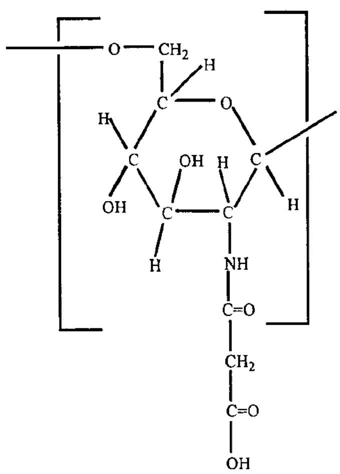
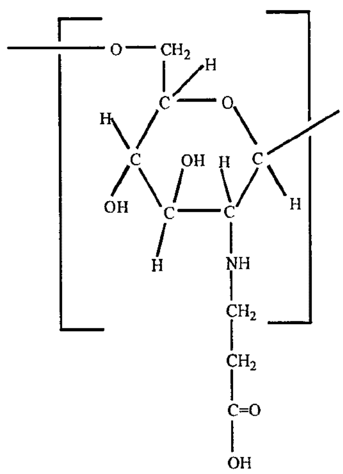

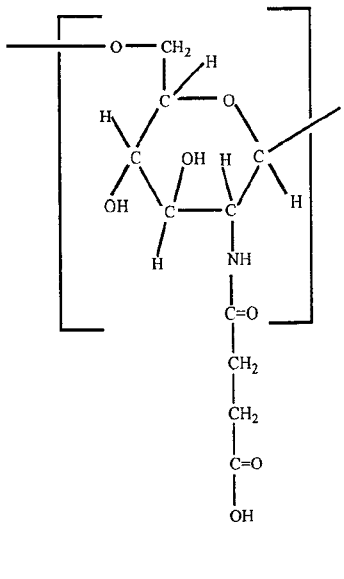

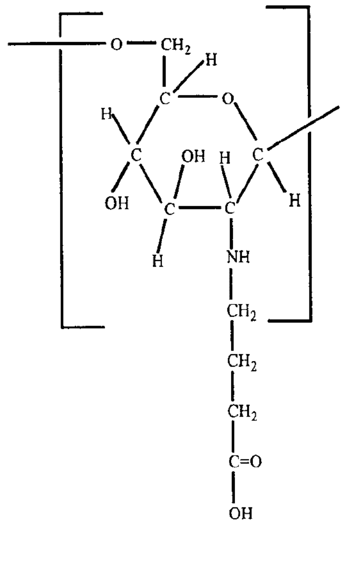

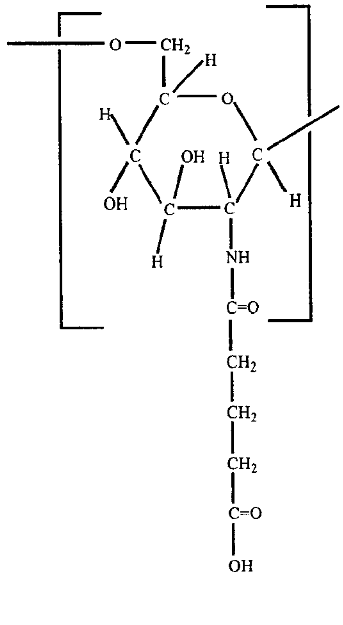

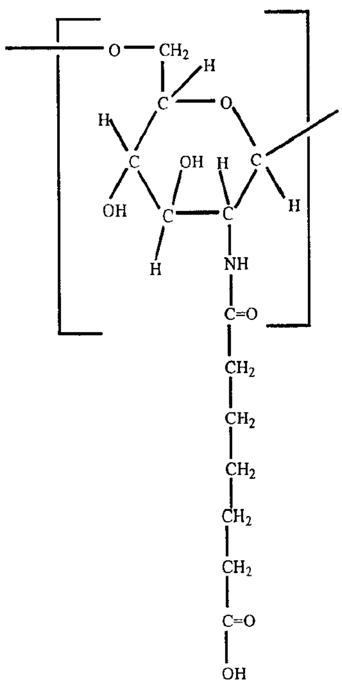

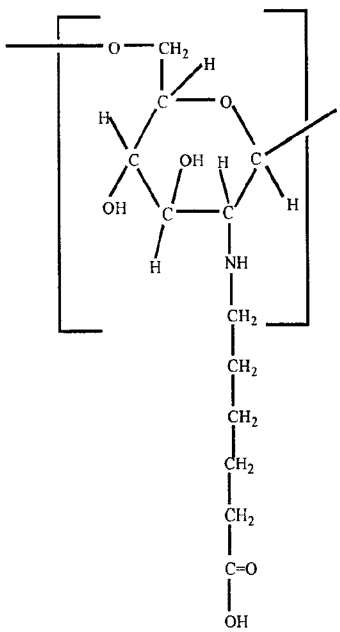

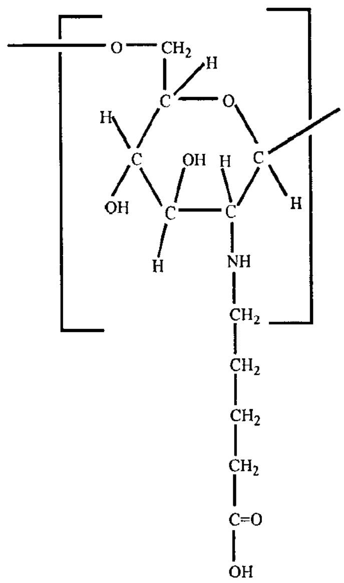

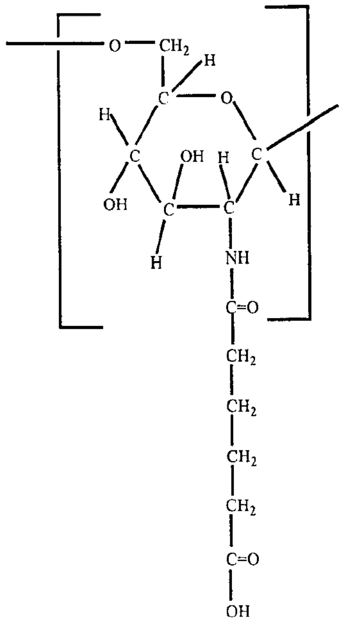

The invention according to another aspect includes synthetic PS/A which differs from the native antigen in the degree and type of substitution. The native PS/A antigen is approximately 100% substituted with succinic acid (1 glucosamine: 1 succinic acid). One type of PS/A antigen according to the invention includes an antigen in which between 10 and 95% of the glucosamine residues are substituted with succinic acid. The invention also includes PS/A antigens having between 10 and 100% substitution with a short chain fatty acid other than succinic acid or a mixture of that short chain fatty acid and succinic acid.

The native PS/A antigen is a high molecular weight homopolymer of greater than 100,000 Daltons. The composition of the invention, however, includes low molecular weight and high molecular weight homopolymers of substituted monomers. The size of the polysaccharides useful according to the invention varies greatly. Polysaccharides between 500 and 20,000,000 daltons will be typical. The polysaccharides of smaller molecular weight may be conjugated to carriers when used as therapeutic or diagnostic agents or may be used alone. Preferably, the polysaccharide composition of the invention has a molecular weight of at least 25,000 Daltons and more preferably at least 30,000 Daltons.

The native material includes glucosamine monomers linked together by a β-1-6 linkage. The polysaccharides of the invention, however, include monomers linked together by either an α- or a β- 1-6, 1-4, or 1-3 linkage or a combination thereof. In a preferred embodiment the polysaccharide is linked together by a β-1-6 linkage.

The compositions of the invention can be isolated from natural sources or synthesized. The invention includes methods for isolating and purifying PS/A antigen from *staphylococci.* One method of the invention includes the steps of extracting a crude PS/A preparation from a bacterial culture, isolating a high molecular weight PS/A-enriched material from the crude PS/A preparation, precipitating an impure PS/A from the high molecular weight PS/A-enriched material, incubating the impure PS/A with a base to produce a semi-pure PS/A preparation, neutralizing the preparation with an acid, and incubating the neutralized preparation in hydrofluoric acid to produce the pure PS/A. The steps of extracting the crude PS/A preparation and isolating and precipitating the impure PS/A antigen preparation are performed by any methods known in the art, such as those including U.S. Patent No. 5,055,455. This impure material is then purified to produce the composition of the invention. The purification steps are achieved by incubating the impure PS/A antigen with a base, such as NaOH, followed by neutralization with an acid such as HCL. The neutral material is then incubated in hydrofluoric acid to produce a pure PS/A antigen of the invention. Another method of the invention includes the steps of extracting a crude antigen suspension from a bacterial culture by incubating the bacteria with a strong base or acid. Preferably, the bacterial is stirred in the strong base or acid for at least 2 hours, and more preferably at least 5,10, 15,18 or 24 hours. The strong base or acid can be any type of strong base or acid, but is preferably greater than 1 Molar NaOH or HCL. In some embodiments the strong base or acid is 5 Molar NaOH or HCL. The acid or base solution is then subjected to centrifugation to collect the cell bodies. In some embodiments the extraction procedure is repeated several times. The resultant acid or base solution is neutralized to pH 7 and then dialyzed to produce an insoluble crude antigen. PS/A can be purified from any bacterial strains expressing the *ica* locus. These strains include, for example, *S. epidermis, S. aureus,* and other strains such as *S. carnosus* which have been transformed with the genes in the *ica* locus. In

particular, pure PS/A has been isolated and purified from specific strains including *S. epidermis* RP62A (APCC number: 35984), *S. epidermis* RP12 (APCC number: 35983), *S. epidermis* M187, S. *carnosis* TM300 (pCN27), *S. aureus* RN4220 (pCN27), and *S. aureus* MN8 mucoid.

The PS/A useful according to the invention also may be synthesized from naturally occurring polysaccharides that do not possess the substituted monomeric unit of native PS/A. For instance, the PS/A antigen of the invention may be synthesized by chemically modifying a polymer of polyglucosamine-like monomeric residues such that those residues are substituted with succinic acid and/or a short chain fatty acid. The resultant polysaccharide is a polymer of monomeric repeating units selected from the group consisting of β 1-6, α 1-6, α 1-4, α 1-3, β 1-4, and β 1-3 polyglucose having the substitution on the C2 atom of the polyglucose. For example, certain naturally occurring polysaccharides have repeating units of an N-acetyl moiety on a polyglucose monomer. Such polysaccharides may be de-N-acetylated to convert the N-acetyl moiety to a free amino moiety, which can be derivitized with a the fatty acid structure of the formulas provided above, thereby creating the necessary structural motif. Other naturally occurring polysaccharides include imine groups which can be reduced to form a free amino moiety, which can be derivitized, thereby creating the necessary structural motif.

Compounds which are useful for these synthetic reactions are known to those of skill in the art in view of the PS/A structure disclosed herein and include, but are not limited to, chitin, chitosan, polyglucose (i.e., dextran), polyglucosamine, and polyglucosaminouronic acid. Chitin for example is a poly-N-acetyl glucosamine with β-1-4 linkages, having the following structure:

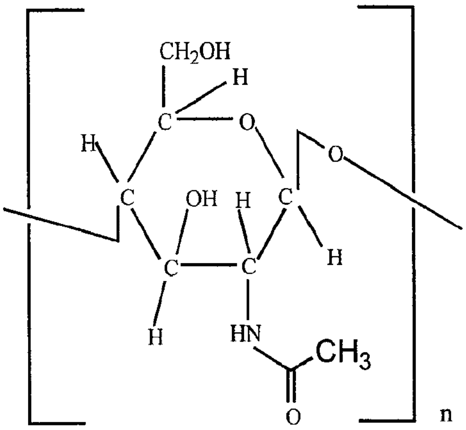

The N-acetyl groups of chitin can be readily removed by treatment with a base, such as 1.0 - 5.0 M NaOH at 95 °C, for 1 hour, to yield chitosan, which is a polyglucosamine with β-1-4 linkages having the following structure:

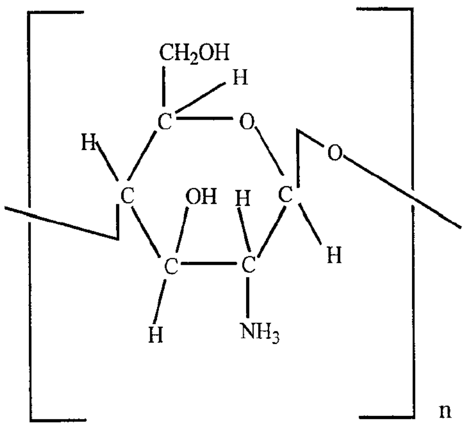

The free amino groups of this compound can then be substituted with succinate or any other short chain fatty acid by incubating the acid with the chitosan material in the presence of a water soluble carbodiimide reagent that promotes the coupling of free amino groups to free carboxyl groups.

Dextran is a polyglucose molecule with α-1-6 linkages, having the following structure:

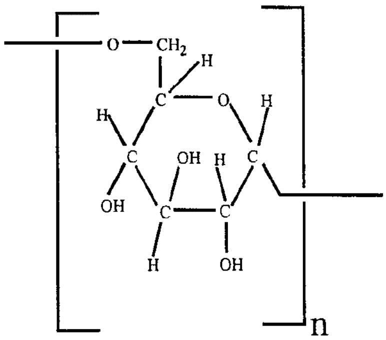

Polyglucosamines having various substituents which are not encompassed by the PS/A antigen described may also be modified to produce the PS/A antigen of the invention. For instance polysaccharide intercellular adhesin (PI/A) is a polymer of β-1-6 linked glucosamine residues substituted on the amine group with an acetate. The acetate can be removed to produce a free amine group which can then be substituted to produce the PS/A of the invention. PI/A has the following structure:

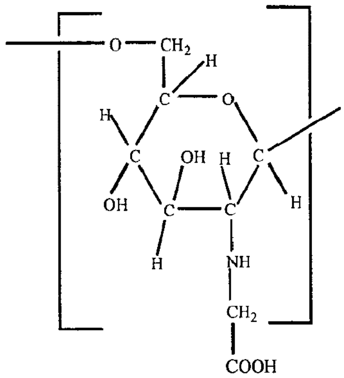

Polyglucosaminouronic acid may also be modified to produce the PS/A antigen of the invention. Polyglucosaminouronic acid may be synthesized or isolated from native sources.

In other embodiments polygalactosaminouronic acid may also be modified to produce a PS/A antigen of the invention. For instance the capsular polysaccharide (Vi antigen) of *Salmonella typhi* is formed entirely of repeating α-1-4 linked monomers of galactosaminuronic acid. This acid includes an N-acetyl moiety that can be modified to yield a free amino group which can be modified to add the substituent of PS/A. The carboxyl group of the uronic acid may also be modified to a $CH_2OH$ group , which is easily accomplished with a water soluble carbodiimide (described in Example 2). The isolation and preparation of *Salmonella typhi* capsule Vi antigen is described in Szu, S.C., X. Li, A.L. Stone and J.B. Robbins, Relation between structure and immunologic properties of the Vi capsular polysaccharide, Infection and Immunity. 59:4555-4561 (1991). The Vi antigen has the following structure:

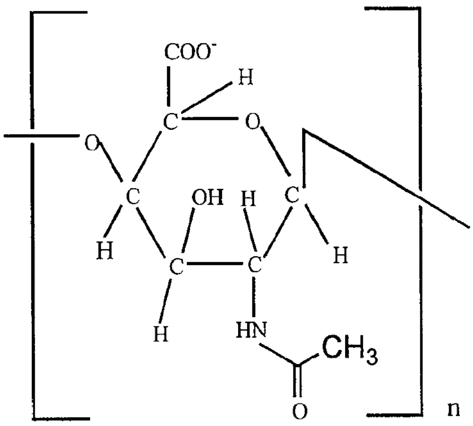

In general de-N-acetylation can be accomplished by conventional chemistry techniques well known to those of ordinary skill in the art. One suitable method involves the use of alkali with or without sodium borohydride. Twenty mg of polysaccharide is dissolved in 2M NaOH (3ml) and sodium borohydride is added (50 mg). The solution is heated to 100oC for 5 h. Following neutralization with acid, the solution is dialyzed against distilled water in the cold and freeze-dried. DiFabio, J. L, Michon, F., Brisson, J.R., Jennings, H.J., Wessels, M.R. Benedi, V.J., Kasper, D.L. Structure of the capsular polysaccharide antigen of type IV groups B Streptococcus. 1989. Canadian Journal of Chemistry, 67:877-882.

For those polysaccharides that contain imine moieties (C-NH), free amino groups can be formed by conventional chemistry techniques known to those of ordinary skill in the art. One suitable method involves the use of sodium borohydride. The imine group can be reduced with sodium borohydride to create a free amino group. This is done by adding in excess of 5 mg of borohydride to polysaccharide dissolved in distilled water while stirring at room temperature for 2 hours. The mixture is then dialyzed against water and freeze dried. DiFabio, J. L et al., *Id.*

In one aspect the invention is a composition of a polymer containing positively (amino) and negatively (fatty acid such as succinyl) charged groups when the fatty acid groups, e.g., succinyl are partially removed from the amino groups such that the molecule is not fully N-succinylated. The polymer has a polyglucosamine backbone, as described above, and is partially substituted with a short fatty acid group, also described above. The other backbone monomers that are not substituted with a short chain fatty acid group are substituted with an amine. The amine is positively charged and the fatty acid is negatively charged. The result is a polymer substituted with some negative charge and some positive charge. The charges may be in blocks. For instance 100 consecutive monomers may have an amino substitution and 100 consecutive monomers may have a fatty acid substitution. Alternatively, the charges may be intermixed randomly or in repetitive patterns or every other monomer may have an amino group and be situated between monomers having a fatty acid group.

The compositions of the invention are useful in a variety of different applications including *in vitro, in situ* and *in vivo* diagnosis of pathological status, such as infection. Additionally, these compositions may be used to immunize subjects *in vivo* to prevent infection. The compositions may also be used to develop antibodies and other binding peptides which are useful for the same purposes as the PS/A compositions of the invention. Thus, the invention includes methods for generating antibodies which are specific for PS/A and which can be used in the diagnosis and treatment of infectious disorders.

The antibodies useful according to the invention may be either monoclonal antibodies or polyclonal antibodies. Polyclonal antibodies generally are raised in animals by multiple subcutaneous or intraperitoneal injections of an antigen and an adjuvant. Polyclonal antibodies to PS/A antigen can be generated by injecting the PS/A antigen alone or in combination with an adjuvant, or by injecting PS/A conjugated to a carrier compound. For instance, it may be useful to conjugate the PS/A antigen to a protein, e.g., keyhole limpet hemocyanin, serum albumin, bovine thyroglobulin, or soy bean trypsin inhibitor, that is immunogenic in the species of animal to be immunized.

Many methods are known in the art for conjugating a polysaccharide to a protein. In general, the polysaccharide should be activated or otherwise rendered amenable to conjugation, i.e., at least one moiety must be rendered capable of covalently bonding to a protein or other molecule. Many such methods are known in the art. For instance, U.S. Patent No. 4,356,170, issued to Jennings, describes the use of periodic acid to generate aldehyde groups on the polysaccharide and then performs reductive amination using cyanoborohydride. U.S. Patent No. 4,663,160, issued to Tsay et al., also used periodic acid to generate aldehyde groups but then linked the polysaccharide to a protein derivatized with a 4-12 carbon moiety (prepared in the presence of a condensing agent) with a Schiff's base reaction in the presence of a reducing agent such as cyanoborohydride. U.S. Patent No. 4,619,828, issued to Gordon, used cyanogen bromide to active the polysaccharide and then conjugated it through a spacer bridge of 4-8 carbon atoms to the protein. In U.S.

Patent No. 4,808,700, issued to Anderson and Clements, a polysaccharide was modified to produce at least one reducing end using limited oxidative cleavage by periodate, hydrolysis by glycosidases, or acid hydrolysis and was conjugated to a protein through reductive amination in the presence of cyanoborohydride. U.S. Patent No. 4,711,779, issued to Porro and Costantino, described the activation of polysaccharides by introducing primary amino groups into the terminal reducing group using sodium cyanoborohydride, followed by conversion to esters in the presence of adipic acid derivatives and conjugation to a toxoid in the presence of an organic solvent, such as dimethylsulfoxide. Many other methods of conjugation are known in the art.

The carrier compound may be directly linked to the PS/A antigen or may be connected to the PS/A antigen through a linker or spacer. A polysaccharide may be coupled to a linker or a spacer by any means known in the art, for example, using a free reducing end of the polysaccharide to produce a covalent bond with a spacer or linker. A covalent bond may be produced by converting a free reducing end of a PS/A antigen into a free 1-aminoglycocide, which can subsequently be covalently linked to a spacer by acylation. (Lundquist et al., J. Carbohydrate Chem., 10:377 (1991)). Alternatively, the PS/A antigen may be covalently linked to the spacer using an N-hydroxysuccinimide active ester as activated group on the spacer. (Kochetkow, Carbohydrate Research, 146:C1 (1986)). The free reducing end of the PS/A antigen may also be converted to a lactone using iodine and potassium hydroxide. (Isebell et al., Methods of Carbohydrate Chemistry, Academic Press, New York (1962)). The lactone can be covalently linked to the spacer by means of a primary amino group on the spacer or linker. The free reducing end of the PS/A antigen may also be covalently linked to the linker or spacer using reductive amination.

The carrier compound linked to the PS/A antigen may be an immunologically active or inert protein. Proteins include, for example, plasma proteins such as serum albumin, immunoglobulins, apolipoproteins and transferrin, bacterial polypeptides such as TRPLE, β-galactosidase, polypeptides such as herpes gD protein, allergins, diphtheria and tetanus toxoids, salmonella flagellin, hemophilus pilin, hemophilus 15kDa, 28-30kDa, and 40kDa membrane proteins, *escherichia coli,* heat label enterotoxin ltb, cholera toxin, and viral proteins including rotavirus VP and respiratory syncytial virus f and g proteins. The proteins which are useful according to the invention include any protein which is safe for administration to mammals and which serves as an immunologically effective carrier protein.

To prepare the polyclonal antibodies the PS/A antigen or antigen conjugate generally is combined with Freund's complete adjuvant or other adjuvant (1 mg or μg of conjugate for rabbits or mice, respectively, in 3 volumes of Freund's) and injected intradermally at multiple sites. Approximately one month later, the animals are boosted with 1/5 - 1/10 of the original amount of antigen or antigen conjugate in Freund's complete adjuvant by subcutaneous injection at multiple sites. One to two weeks later the animals are bled, and the serum is assayed for the presence of antibody. The animals may be repeatedly boosted until the antibody titer plateaus. The animal may be boosted with the PS/A antigen alone, the PS/A antigen conjugate, or PS/A conjugated to a different carrier compound. An aggregating agent, such as alum, can also be used to enhance the immune response.

In addition to supplying a source of polyclonal antibodies, the immunized animals can be used to generate PS/A antigen specific monoclonal antibodies. As used herein the term "monoclonal antibody" refers to a homogenous population of immunoglobulins which specifically bind to an epitope (i.e. antigenic determinant) of PS/A. Monoclonal antibodies can be prepared by any method known in the art such as by immortalizing spleen cells isolated from the immunized animal by e.g., fusion with myeloma cells or by Epstein-bar-virus transformation and screening for clones expressing the desired antibody. Methods for preparing and using monoclonal antibodies are well known in the art.

Murine anti-PS/A monoclonal antibodies may be made by any of these methods utilizing PS/A as an immunogen. The following description of a method for developing an anti-PS/A monoclonal antibody is exemplary and is provided for illustrative purposes only. Balb/c mice are immunized intraperitoneally with approximately 75-100 μg of purified PS/A in an complete Freund's adjuvant. Booster injections of approximately 25-50 μg PS/A in incomplete Freund's are administered on approximately days 15 and 35 after the initial injection. On day 60-65, the mice receive booster injections of approximately 25 μg PS/A in the absence of adjuvant. Three days later, the mice are killed and the isolated spleen cells fused to murine myeloma NS-1 cells using polyethylene glycol by a procedure such as that described by Oi (Oi VT: Immunoglobulin-producing hybrid cell lines in Hezenberg LA (ed): selected methods in cellular biology, San Francisco, CA, Freeman, (1980)). Hybridoma cells are selected using hypoxanthine, aminopterin, and thymidine and grown in culture. Fourteen to fifteen days after fusion, hybridoma cells producing anti-PS/A monoclonal antibodies are identified using a solid-phase radioimmunoassay by capturing anti-PS/A antibodies from conditioned media with immobilized goat anti-mouse IgG followed by quantitation of specifically bound $^{125}$I-labeled PS/A. Hybridomas testing positive for antibodies against PS/A are subcloned by limiting dilution and retested. Ascites for the hybridomas is then prepared in pristane-primed BALB/c mice by injecting approximately $1x10^6$ cells/mouse. Concentrates enriched in the selected monoclonal antibodies are produced from ascites fluid by gel filtration on S-200 and concentrated with $(NH_4)SO_4$. The pellets are dissolved in an appropriate storage solution such as 50% glycerol/$H_2O$ and are stored at 4°C.

An "anti-PS/A antibody" as used herein includes humanized antibodies and antibody fragments as well as intact monoclonal and polyclonal antibodies.

In preferred embodiments, the anti-PS/A antibody useful according to the methods of the invention is an intact

humanized anti-PS/A monoclonal antibody in an isolated form or in a pharmaceutical preparation. A "humanized monoclonal antibody" as used herein is a human monoclonal antibody or functionally active fragment thereof having human constant regions and a PS/A binding region from a mammal of a species other than a human.

Human monoclonal antibodies may be made by any of the methods known in the art, such as those disclosed in US Patent No. 5,567,610, issued to Borrebaeck et al., US Patent No. 565,354, issued to Ostberg, US Patent No. 5,571,893, issued to Baker et al, Kozber, J. Immunol. 133: 3001 (1984), Brodeur, et al., Monoclonal Antibody Production Techniques and Applications, p. 51-63 (Marcel Dekker, Inc, new York, 1987), and Boerner el al., J. Immunol., 147: 86-95 (1991). In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immuno., 7: 33 (1993) and US Patent No. 5,569,825 issued to Lonberg).

The following examples of methods for preparing humanized monoclonal antibodies that interact with PS/A are exemplary and are provided for illustrative purposes only. Humanized monoclonal antibodies, for example, may be constructed by replacing the non-CDR regions of a non-human mammalian antibody with similar regions of human antibodies while retaining the epitopic specificity of the original antibody. For example, non-human CDRs and optionally some of the framework regions may be covalently joined to human FR and/or Fc/pFc' regions to produce a functional antibody. There are entities in the United States which will synthesize humanized antibodies from specific murine antibody regions commercially, such as Protein Design Labs (Mountain View California).

European Patent Application 0239400, the entire contents of which is hereby incorporated by reference, provides an exemplary teaching of the production and use of humanized monoclonal antibodies in which at least the CDR portion of a murine (or other non-human mammal) antibody is included in the humanized antibody. Briefly, the following methods are useful for constructing a humanized CDR monoclonal antibody including at least a portion of a mouse CDR. A first replicable expression vector including a suitable promoter operably linked to a DNA sequence encoding at least a variable domain of an Ig heavy or light chain and the variable domain comprising framework regions from a human antibody and a CDR region of a murine antibody is prepared. Optionally a second replicable expression vector is prepared which includes a suitable promoter operably linked to a DNA sequence encoding at least the variable domain of a complementary human Ig light or heavy chain respectively. A cell line is then transformed with the vectors. Preferably the cell line is an immortalized mammalian cell line of lymphoid origin, such as a myeloma, hybridoma, trioma, or quadroma cell line, or is a normal lymphoid cell which has been immortalized by transformation with a virus. The transformed cell line is then cultured under conditions known to those of skill in the art to produce the humanized antibody.

As set forth in European Patent Application 0239400 several techniques are well known in the art for creating the particular antibody domains to be inserted into the replicable vector. (Preferred vectors and recombinant techniques are discussed in greater detail below.) For example, the DNA sequence encoding the domain may be prepared by oligonucleotide synthesis. Alternatively a synthetic gene lacking the CDR regions in which four framework regions are fused together with suitable restriction sites at the junctions, such that double stranded synthetic or restricted subcloned CDR cassettes with sticky ends could be ligated at the junctions of the framework regions. Another method involves the preparation of the DNA sequence encoding the variable CDR containing domain by oligonucleotide site-directed mutagenesis. Each of these methods is well known in the art. Therefore, those skilled in the art may construct humanized antibodies containing a murine CDR region without destroying the specificity of the antibody for its epitope.

Humanized antibodies have particular clinical utility in that they specifically recognize PS/A but will not evoke an immune response in humans against the antibody itself. In a most preferred embodiment, a murine CDR is grafted into the framework region of a human antibody to prepare the "humanized antibody." See, e.g., L. Riechmann et al., Nature 332, 323 (1988); M. S. Neuberger et al., Nature 314, 268 (1985) and EPA 0 239 400 (published Sep. 30, 1987).

PS/A binding antibody fragments are also encompassed by the invention. As is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions of the antibody, for example, are effectors of the complement cascade but are not involved in antigen binding. An antibody from which the pFc' region has been enzymatically cleaved, or which has been produced without the pFc' region, designated an $F(ab')_2$ fragment, retains both of the antigen binding sites of an intact antibody. An isolated $F(ab')_2$ fragment is referred to as a bivalent monoclonal fragment because of its two antigen binding sites. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd (heavy chain variable region). The Fd fragments are the major determinant of antibody specificity (a single Fd fragment may be associated with up to ten different light chains without altering antibody specificity) and Fd fragments retain epitope-binding ability in isolation.

The terms Fab, Fc, pFc', $F(ab')_2$ and Fv are employed with either standard immunological meanings [Klein,

Immunology (John Wiley, New York, NY, 1982); Clark, W.R. (1986) The Experimental Foundations of Modern Immunology (Wiley & Sons, Inc., New York); Roitt, 1. (1991) Essential Immunology, 7th Ed., (Blackwell Scientific Publications, Oxford)]. Well-known functionally active antibody fragments include but are not limited to $F(ab')_2$, Fab, Fv and Fd fragments of antibodies. These fragments which lack the Fc fragment of intact antibody, clear more rapidly from the circulation, and may have less non-specific tissue binding than an intact antibody (Wahl et al., J. Nucl. Med. 24:316-325 (1983)). For example, single-chain antibodies can be constructed in accordance with the methods described in U.S. Patent No. 4,946,778 to Ladner et al. Such single-chain antibodies include the variable regions of the light and heavy chains joined by a flexible linker moiety. Methods for obtaining a single domain antibody ("Fd") which comprises an isolated variable heavy chain single domain, also have been reported (see, for example, Ward et al., Nature 341:644-646 (1989), disclosing a method of screening to identify an antibody heavy chain variable region ($V_H$ single domain antibody) with sufficient affinity for its target epitope to bind thereto in isolated form). Methods for making recombinant Fv fragments based on known antibody heavy chain and light chain variable region sequences are known in the art and have been described, e.g., Moore et al., US Patent No. 4,462,334. Other references describing the use and generation of antibody fragments include e.g., Fab fragments (Tijssen, Practice and Theory of Enzyme Immunoassays (Elsevieer, Amsterdam, 1985)), Fv fragments (Hochman et al., Biochemistry 12: 1130 (1973); Sharon et al., Biochemistry 15: 1591 (1976); Ehrilch et al., U.S. Patent No. 4,355,023) and portions of antibody molecules (Audilore-Hargreaves, U.S. patent No. 4,470,925). Thus, those skilled in the art may construct antibody fragments from various portions of intact antibodies without destroying the specificity of the antibodies for the PS/A epitope.

The antibody fragments of the invention also encompass "humanized antibody fragments." As one skilled in the art will recognize, such fragments could be prepared by traditional enzymatic cleavage of intact humanized antibodies. If, however, intact antibodies are not susceptible to such cleavage, because of the nature of the construction involved, the noted constructions can be prepared with immunoglobulin fragments used as the starting materials; or, if recombinant techniques are used, the DNA sequences, themselves, can be tailored to encode the desired "fragment" which, when expressed, can be combined *in vivo* or *in vitro,* by chemical or biological means, to prepare the final desired intact immunoglobulin fragment.

Antibody fragments and other PS/A binding polypeptides having binding specificity for PS/A for the diagnostic methods of the invention set forth below also are embraced by the invention. Several routine assays may be used to easily identify such peptides. Screening assays for identifying peptides of the invention are performed for example, using phage display procedures such as those described in Hart, et al., J. Biol. Chem. 269:12468 (1994). Hart et al. report a filamentous phage display library for identifying novel peptide ligands for mammalian cell receptors. In general, phage display libraries using, e.g., M13 or fd phage, are prepared using conventional procedures such as those described in the foregoing reference. The libraries display inserts containing from 4 to 80 amino acid residues. The inserts optionally represent a completely degenerate or a biased array of peptides. Ligands that bind selectively to PS/A are obtained by selecting those phages which express on their surface a ligand that binds to PS/A. These phages then are subjected to several cycles of reselection to identify the peptide ligand-expressing phages that have the most useful binding characteristics. Typically, phages that exhibit the best binding characteristics (e.g., highest affinity) are further characterized by nucleic acid analysis to identify the particular amino acid sequences of the peptides expressed on the phage surface and the optimum length of the expressed peptide to achieve optimum binding to PS/A. Alternatively, such peptide ligands can be selected from combinatorial libraries of peptides containing one or more amino acids. Such libraries can further be synthesized which contain non-peptide synthetic moieties which are less subject to enzymatic degradation compared to their naturally-occurring counterparts.

To determine whether a peptide binds to PS/A any known binding assay may be employed. For example, the peptide may be immobilized on a surface and then contacted with a labeled PS/A. The amount of PS/A which interacts with the peptide or the amount which does not bind to the peptide may then be quantitated to determine whether the peptide binds to PS/A. A surface having an anti-PS/A antibody immobilized thereto may serve as a positive control.

The compositions of the invention are useful for many *in vivo,* and *in vitro* purposes. For example, the compositions of the invention are useful for producing an antibody response, e.g., as a vaccine for active immunization of humans and animals to prevent *S. aureus* infection and infections caused by other species of bacteria that make the PS/A antigen; as a vaccine for immunization of humans or animals to produce anti-PS/A antibodies that can be administered to other humans or animals to prevent or treat staphylococcal infections; as an antigen to screen for important biological agents such as monoclonal antibodies capable of preventing *S. aureus* infection, libraries of genes involved in making antibodies, or peptide mimetics; as a diagnostic reagent for *S. aureus* infections and infections caused by other species of bacteria that make the PS/A antigen; and as a diagnostic reagent for determining the immunologic status of humans or animals in regard to their susceptibility to *S. aureus* infections and infections caused by other species of bacteria that make the PS/A antigen.

The PS/A of the invention can be used to protect a subject against infection with a bacteria which has a PS/A capsule on its surface by inducing active immunity to infection by *staphylococci* in a subject. The method is accomplished by administering to the subject an effective amount for inducing active immunity to *staphylococci* of any of the PS/A

compositions of the invention described above.

"Active immunity" as used herein involves the introduction of an antigen into a subject such that the antigen causes differentiation of some lymphoid cells into cells that produce antibody and other lymphoid cells into memory cell. The memory cells do not secrete antibodies but rather incorporate the antibodies into their membrane in order to sense antigen if it is administered to the body again.

The method is useful for inducing immunity to infection by *staphylococci. "Staphylococci"* as used herein refers to all *staphylococcal* bacterial species expressing a PS/A containing capsule. Bacteria which are classified as *staphylococci* are well known to those of skill in the art and are described in the microbiology literature. *Staphylococci* expressing a PS/A containing capsule include but are not limited *Staphylococcus epidermidis* (including RP62A (ATCC Number: 35984), RP12 (ATCC Number: 35983), and M187), *Staphylococcus aureus* (including RN4220 (pCN27) and MN8 mucoid), and strains such as *Staphylococcus carnosus* transformed with the genes in the *ica* locus (including TM300 (pCN27)). Other bacterial strains expressing a PS/A containing capsule can easily be identified by those of skill in the art. For instance a *staphylococcal* bacteria which expresses the *ica* locus will express a PS/A containing capsule. One of ordinary skill in the art can easily screen for the expression of mRNA or protein related of the *ica* locus since the nucleic acid sequence of the *ica* locus is known (SEQ ID NO. 1 and originally described in Heilmann, C., O. Schweitzer, C. Gerke, N. Vanittanakom, D. Mack and F. Gotz (1996) Molecular basis of intercellular adhesion in the biofilm-forming Staphylococcus epidermidis. Molec. Microbiol. 20:1083.*)* Although the Heilmann publication describes the nucleic acid sequence of the *ica* locus, the publication erroneously states that this locus encodes production of the PIA antigen. It was discovered according to the instant invention that *ica* actually encodes for proteins involved in the production of PS/A. Bacterial strains expressing a PS/A containing capsule also can be identified by immunoelectron microscopy (or other immunoassay) using anti-PS/A antibodies to detect the presence of the capsule on the surface of the bacteria as described in Example 6 below. Additionally the capsule of bacterial strains can be isolated as described in Example 1 and analyzed using liquid chromatography and mass spectroscopy as described in Example 4 below.

A "subject" as used herein is a warm-blooded mammal and includes, for instance, humans, primates, horses, cows, swine, goats, sheep, chicken, dogs, and cats.

The PS/A of the invention may be administered to any subject capable of inducing an immune response to an antigen but are especially adapted to induce active immunization against systemic infection caused by *staphylococci* in a subject capable of inducing an immune response and at risk of developing a *staphylococcal* infection. A subject capable of inducing an immune response and at risk of developing a *staphylococcal* infection is a mammal possessing a normal healthy immune system that is at risk of being exposed to environmental *staphylococci.* For instance, a hospitalized patient which is not otherwise immunocompromised is at risk of developing staphylococcal infection as a result of exposure to the bacteria in the hospital environment. In particular high risk populations for developing infection by *S. aureus* include, for example, renal disease patients on dialysis, and individuals undergoing high risk surgery. High risk populations for developing infection by *S. epidermidis* include, for example, patients with indwelling medical devices because clinical isolates are often highly adherent to plastic surfaces as a result of their extracellular material referred to as biofilm or slime.

The PS/A of the invention is administered to the subject in an effective amount for inducing an antibody response. An "effective amount for inducing an antibody response" as used herein is an amount of PS/A which is sufficient to (i) assist the subject in producing its own immune protection by e.g. inducing the production of anti-PS/A antibodies in the subject, inducing the production of memory cells, inducing a cytotoxic lymphocyte reaction etc. and/or (ii) prevent infection by *staphylococci* from occurring in a subject which is exposed to *staphylococci.*

One of ordinary skill in the art can assess whether an amount of PS/A is sufficient to induce active immunity by routine methods known in the art. For instance the ability of a specific antigen to produce antibody in a mammal can be assessed by screening the PS/A antigen in a mouse or other subject.

A preferred method of the invention is a method for inducing active immunity to infection specifically by *staphylococcus aureus* in a subject. Prior to the instant invention it was not known that *staphylococcus aureus* expresses PS/A on its surface. It was believed that CP5 and CP8 made up the external capsule of *staphylococcus aureus* and that these capsules might be effective antigens for inducing immunity. It was discovered according tot he invention, however, that PS/A is useful as an antigen in inducing immunity active immunity to infection by *staphylococcus aureus.* The invention therefore includes the method of inducing immunity by administering to a subject a high molecular weight polysaccharide having a polyglucosamine backbone wherein at least 10% of individual glucosamine units of the polyglucosamine backbone are conjugated to succinate through the amine of the C2 atom.

The anti-PS/A antibodies of the invention are useful for inducing passive immunization in a subject by preventing the development of systemic infection in those subjects at risk of exposure to infectious agents. The method for inducing passive immunity to infection by *staphylococcus aureus* is performed by administering to a subject an effective amount for inducing opsonization of *staphylococcus aureus* of an anti-PS/A antibody.

"Passive immunity" as used herein involves the administration of antibodies to a subject, wherein the antibodies are produced in a different subject (including subjects of the same and different species), such that the antibodies attach

to the surface of the bacteria and cause the bacteria to be phagocytized.

The anti-PS/A antibody of the invention may be administered to any subject at risk of developing a *staphylococcal* infection to induce passive immunity. The anti-PS/A antibody can even be administered to a subject that is incapable of inducing an immune response to an antigen. Although vaccination with a PS/A antigen might not be effective in high risk immunocompromised subjects, these subjects will benefit from treatment with antibody preparations raised against *S. aureus.* A subject that is incapable of inducing an immune response is an immunocompromised subject (e.g. patient undergoing chemotherapy, AIDS patient, etc.) or a subject that has not yet developed an immune system (e.g. preterm neonate). The anti-PS/A antibody may be administered to a subject at risk of developing a *staphylococcal* infection to prevent the infectious agent from multiplying in the body or to kill the infectious agent. The anti-PS/A antibody may also be administered to a subject who already has an infection caused by *staphylococci* to prevent the infectious agent from multiplying in the body or to kill the infectious agent.

The anti-PS/A antibody of the invention is administered to the subject in an effective amount for inducing an immune response to *staphylococcus aureus.* An "effective amount for inducing an immune response to *staphylococcus aureus"* as used herein is an amount of PS/A which is sufficient to (i) prevent infection by *staphylococci* from occurring in a subject which is exposed to *staphylococci;* (ii) inhibit the development of infection, i.e., arresting or slowing its development; and/or (iii) relieve the infection, i.e. eradication of the bacteria causing the infection.

Using routine procedures known to those of ordinary skill in the art, one can determine whether an amount of anti-PS/A antibody is an "effective amount for inducing an immune response to *staphylococcus aureus"* in an *in vitro* opsonization assay which is predictive of the degree of opsonization of an antibody. An antibody which opsonizes a *staphylococcal* bacteria is one which when added to a sample of *staphylococcal* bacteria causes phagocytosis of the bacteria. An opsonization assay may be a colorimetric assay, a chemiluminescent assay, a fluorescent or radiolabel uptake assay,, a cell mediated bactericidal assay or other assay which measures the opsonic potential of a material. For instance, the following opsonization assay may be used to determine an effective amount of anti-PS/A antibody. The anti-PS/A antibody is incubated with an *staphylococcal* bacteria and a eukaryotic phagocytic cell and optionally complement proteins. The opsonic ability of the anti-PS/A antibody is determined based on the amount of *staphylococci* that remain after incubation. This can be accomplished by comparing the number of surviving *staphylococci* between two similar assays, only one of which includes opsonizing immunoglobulin or by measuring the number of viable *staphylococci* before and after the assay. A reduction in the number of *staphylococci* indicates opsonization.

The methods of the invention are also useful for inducing passive immunization to coagulase negative *staphylococci* in a subject by administering to a subject an effective amount for inducing opsonization of coagulase negative *staphylococci* of an anti-PS/$A_{pure}$ antibody. Although antibodies directed to PS/A isolated from coagulase negative *staphylococci* have been developed in the prior art and used to induce passive immunity to coagulase negative *staphylococci,* anti-PS/$A_{pure}$ antibodies have not previously been used for this purpose. An, anti-PS/$A_{pure}$ antibody as used herein is an antibody which specifically interacts with a pure PS/A antigen of the invention and induces opsonization of coagulase negative *staphylococci* but which does not interact with the impure preparation of PS/A of the prior art. As discussed above the impure PS/A preparation of the prior art was contaminated with teichoic acid which interfered with the immunogenicity of the antigen. The anti-PS/$A_{pure}$ antibody of the invention does not interact with a PS/A preparation that is contaminated with teichoic acid. One of ordinary skill in the art can easily identify whether an anti-PS/A antibody is an anti-PS/$A_{pure}$ antibody which does not cross-react with an impure PS/A preparation by using routine biding assays. For instance, an anti-PS/A antibody may be immobilized on a surface and then contacted with a labeled impure PS/A preparation or a labeled pure PS/A preparation. The amount of PS/A preparation (pure vs. impure preparation) which interacts with the antibody or the amount which does not bind to the antibody may then be quantitated to determine whether the antibody binds to an impure PS/A preparation. If the antibody interacts with the pure PS/A preparation but does not interact with the impure PS/A preparation then the antibody is an anti-PS/$A_{pure}$ antibody of the invention.

Subjects having a high risk of developing infection by *S. epidermidis* include, for example, preterm neonates, patients undergoing chemotherapy, and other patients with indwelling medical devices. Clinical isolates are often highly adherent to plastic surfaces because they elaborate an extracellular material referred to as biofilm or slime.

When PS/A antigen is used to prevent bacterial infection, it is formulated as a vaccine. A suitable carrier media for formulating a vaccine includes sodium phosphate-buffered saline (pH 7.4) or 0.125 M aluminum phosphate gel suspended in sodium phosphate-buffered saline at pH 6 and other conventional media. Generally, vaccines contain from about 5 to about 100 μg, preferably about 10-50 μg of antigen are suitable to elicit effective levels of antibody against the PS/A antigen in warm-blooded mammals. When administered as a vaccine the PS/A can optionally include an adjuvant.

The term "adjuvant" is intended to include any substance which is incorporated into or administered simultaneously with the PS/A of the invention which potentiates the immune response in the subject. Adjuvants include aluminum compounds, e.g., gels, aluminum hydroxide and aluminum phosphate, and Freund's complete or incomplete adjuvant (in which the PS/A antigen is incorporated in the aqueous phase of a stabilized water in paraffin oil emulsion). The paraffin oil may be replaced with different types of oils, e.g., squalene or peanut oil. Other materials with adjuvant

properties include BCG (attenuated *Mycobacterium tuberculosis),* calcium phosphate, levamisole, isoprinosine, polyanions (e.g., poly A:U), lentinan, pertussis toxin, lipid A, saponins and peptides, e.g. muramyl dipeptide. Rare earth salts, e.g., lanthanum and cerium, may also be used as adjuvants. The amount of adjuvants depends on the subject and the particular PS/A antigen used and can be readily determined by one skilled in the art without undue experimentation.

In general, when administered for therapeutic purposes, the formulations of the invention are applied in pharmaceutically acceptable solutions. Such preparations may routinely contain pharmaceutically acceptable concentrations of salt, buffering agents, preservatives, compatible carriers, adjuvants, and optionally other therapeutic ingredients.

The compositions of the invention may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulphuric, nitric, phosphoric, maleic, acetic, salicyclic, p-toluene sulphonic, tartaric, citric, methane sulphonic, formic, malonic, succinic, naphthalene-2-sulphonic, and benzene sulphonic. Also, pharmaceutically acceptable salts can be prepared as alkaline metal or alkaline earth salts, such as sodium, potassium or calcium salts of the carboxylic acid group.

Suitable buffering agents include: acetic acid and a salt (1-2% W/V); citric acid and a salt (1-3% W/V); boric acid and a salt (0.5-2.5% WN); and phosphoric acid and a salt (0.8-2% W/V). Suitable preservatives include benzalkonium chloride (0.003-0.03% W/V); chlorobutanol (0.3-0.9% W/V); parabens (0.01-0.25% W/V) and thimerosal (0.004-0.02% WN).

The present invention provides pharmaceutical compositions, for medical use, which comprise PS/A of the invention together with one or more pharmaceutically acceptable carriers and optionally other therapeutic ingredients. The term "pharmaceutically-acceptable carrier" as used herein, and described more fully below, means one or more compatible solid or liquid filler, dilutants or encapsulating substances which are suitable for administration to a human or other animal. In the present invention, the term "carrier" denotes an organic or inorganic ingredient, natural or synthetic, with which the active ingredient is combined to facilitate the application. The components of the pharmaceutical compositions also are capable of being commingled with the PS/A antigens of the present invention, and with each other, in a manner such that there is no interaction which would substantially impair the desired pharmaceutical efficiency.

Compositions suitable for parenteral administration conveniently comprise a sterile aqueous preparation of the polysaccharide, which can be isotonic with the blood of the recipient. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di-glycerides. In addition, fatty acids such as oleic acid find use in the preparation of injectables. Carrier formulations suitable for subcutaneous, intramuscular, intraperitoneal, intravenous, etc. administrations may be found in Remington's Pharmaceutical Sciences, Mack Publishing Company, Easton, PA.

The preparations of the invention are administered in effective amounts. An effective amount, as discussed above, is that amount of a PS/A antigen or anti-PS/A antibody that will alone, or together with further doses, induce active immunity or opsonization of the infectious bacteria, respectively. It is believed that doses ranging from 1 nanogram/kilogram to 100 milligrams/kilogram, depending upon the mode of administration, will be effective. The preferred range is believed to be between 500 nanograms and 500 micrograms/kilogram, and most preferably between 1 microgram and 100 micrograms/kilograms. The absolute amount will depend upon a variety of factors including whether the administration is performed on a high risk subject not yet infected with the bacteria or on a subject already having an infection, the concurrent treatment, the number of doses and the individual patient parameters including age, physical condition, size and weight. These are factors well known to those of ordinary skill in the art and can be addressed with no more than routine experimentation. It is preferred generally that a maximum dose be used, that is, the highest safe dose according to sound medical judgment.

Multiple doses of the pharmaceutical compositions of the invention are contemplated. Generally immunization schemes involve the administration of a high dose of an antigen followed by subsequent lower doses of antigen after a waiting period of several weeks. Further doses may be administered as well. The dosage schedule for passive immunization would be quite different with more frequent administration if necessary. Any regimen that results in an enhanced immune response to bacterial infection and/or subsequent protection from infection may be used. Desired time intervals for delivery of multiple doses of a particular PS/A can be determined by one of ordinary skill in the art employing no more than routine experimentation.

A variety of administration routes are available. The particular mode selected will depend, of course, upon the particular PS/A selected, the particular condition being treated and the dosage required for therapeutic efficacy. The methods of this invention, generally speaking, may be practiced using any mode of administration that is medically acceptable, meaning any mode that produces effective levels of an immune response without causing clinically unacceptable adverse effects. Preferred modes of administration are parenteral routes. The term "parenteral" includes subcutaneous injections, intravenous, intramuscular, intraperitoneal, intra sternal injection or infusion techniques.

The compositions may conveniently be presented in unit dosage form and may be prepared by any of the methods well known in the art of pharmacy. All methods include the step of bringing the active PS/A into association with a carrier which constitutes one or more accessory ingredients. In general, the compositions are prepared by uniformly and intimately bringing the polymer into association with a liquid carrier, a finely divided solid carrier, or both, and then, if necessary, shaping the product. The polymer may be stored lyophilized.

Other delivery systems can include time-release, delayed release or sustained release delivery systems. Such systems can avoid repeated administrations of the polysaccharides of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. Specific examples include, but are not limited to: (a) erosional systems in which the polysaccharide is contained in a form within a matrix, found in U.S. Patent Nos. 4,452,775 (Kent); 4,667,014 (Nestor et al.); and 4,748,034 and 5,239,660 (Leonard) and (b) diffusional systems in which an active component permeates at a controlled rate through a polymer, found in U.S. Patent Nos. 3,832,253 (Higuchi et al.) and 3,854,480 (Zaffaroni). In addition, a pump-based hardware delivery system can be used, some of which are adapted for implantation.

It will also be appreciated by those of ordinary skill in the art that the PS/A antigens of the present invention have adjuvant properties by themselves. To the extent that the polysaccharides described herein potentiate human immune responses, they can be used as adjuvants in combination with other materials.

The PS/A antigens and anti-PS/A antibodies of the invention may be delivered in conjunction with another anti-bacterial antibiotic drug or in the form of anti-bacterial, antibiotic cocktails or with other bacterial antigens or antibodies. An anti-bacterial antibiotic cocktail is a mixture of any of a composition useful according to this invention with an anti-bacterial antibiotic drug. The use of antibiotics in the treatment of bacterial infection is routine. The use of antigens for inducing active immunization and antibodies to induce passive immunization is also routine. In this embodiment, a common administration vehicle (e.g., tablet, implant, injectable solution, etc.) could contain both the composition useful in this invention and the anti-bacterial antibiotic drug and/or antigen/antibody. Alternatively, the anti-bacterial antibiotic drug and/or antigen/antibody can be separately dosed.

Anti-bacterial antibiotic drugs are well known and include: penicillin G, penicillin V, ampicillin, amoxicillin, bacampicillin, cyclacillin, epicillin, hetacillin, pivampicillin, methicillin, nafcillin, oxacillin, cloxacillin, dicloxacillin, flucloxacillin, carbenicillin, ticarcillin, avlocillin, mezlocillin, piperacillin, amdinocillin, cephalexin, cephradine, cefadoxil, cefaclor, cefazolin, cefuroxime axetil, cefamandole, cefonicid, cefoxitin, cefotaxime, ceftizoxime, cefmenoxine, ceftriaxone, moxalactam, cefotetan, cefoperazone, ceftazidme, imipenem, clavulanate, timentin, sulbactam, neomycin, erythromycin, metronidazole, chloramphenicol, clindamycin, lincomycin, vancomycin, trimethoprim-sulfamethoxazole, aminoglycosides, quinolones, tetracyclines and rifampin. (See Goodman and Gilman's, Pharmacological Basics of Therapeutics, 8th Ed., 1993, McGraw Hill Inc.)

Other polysaccharide antigens and antibodies are well known in the art. For instance, the following polysaccharide antigens and/or antibodies thereto can be administered in conjunction with the PS/A antigen and /or antibody: *Salmonella typhi* capsule Vi antigen (Szu, S.C., X. Li, A.L. Stone and J.B. Robbins, Relation between structure and immunologic properties of the Vi capsular polysaccharide, Infection and Immunity. 59:4555-4561 (1991)); *E. Coli* K5 capsule (Vann, W., M.A. Schmidt, B. Jann and K. Jann, The structure of the capsular polysaccharide (K5 antigen) of urinary tract infective *Escherichia coli,* 010:K5:H4. A polymer similar to desulfo-heparin, European Journal of Biochemistry. 116: 359-364, (1981)); *Staphylococcus aureus* type 5 capsule (Fournier, J.-M., K. Hannon, M. Moreau, W.W. Karakawa and W.F. Vann, Isolation of type 5 capsular polysaccharide from Staphylococcus aureus, Ann. Inst. Pasteur/ Microbiol. (Paris). 138: 561-567, (1987)); *Rhizobium melilori* expolysaccharide II (Glazebrook, J. and G.C. Walker, a novel expolysaccharide can function in place of the calcofluor-binding exopolysaccharide in nodulation of alfalfa by Rhizobium meliloti, Cell. 65:661-672 (1989)); *Group B streptococcus* type III (Wessels, M.R., V. Pozsgay, D.L. Kasper and H. J. Jennings, Structure and immunochemistry of an oligosaccharide repeating unit of the capsular polysaccharide of type III group B Streptococcus, Journal of Biological Chemistry. 262:8262-8267 (1987)); *Pseudomonas aeruginosa* Fisher 7 O-specific side-chain (Knirel, Y.A., N.A. Paramonov, E.V. Vinogradov, A.S. Shashkow, B.A. N.K. Kochetkov, E.S. Stanislavsky and E.V. Kholodkova, Somatic antigens of Pseudomonas aeruginosa The structure of O-specific polysaccharide chains of lipopolysaccharides of P. aeruginosa 03(Lanyi), 025 (Wokatsch) and Fisher immunotypes 3 and 7, European Journal of Biochemistry. 167:549, (1987)); *Shigella sonnei* O-specific side chain (Kenne, L., B. Lindberg and K. Petersson, Structural studies of the O-specific side-chains of the Shigella sonnei phase I lipopolysaccharide, Carbohydrate Research. 78:119-126, (1980)); *S. pneumoniae* type I capsule (Lindberg, B., Lindqvist, B., Lonngren, J., Powell, D.A., Structural studies of the capsular polysaccharide from Streptococcus pneumoniae type 1, Carbohydrate Research. 78:111-117 (1980)); and *Streptococcus pneumoniae* group antigen (Jennings, H.J., C. Lugowski and N. M. Young, Structure of the complex polysaccharide C-substance from Streptococcus pneumoniae type 1, Biochemistry.

19:4712-4719 (1980)).

Other non-polypeptide antigens and antibodies thereto are well known to the those of skill in the art and can be used in conjunction with the PS/A compositions of the invention.

The PS/A antigens and antibodies are also useful in diagnostic assays for determining an immunologic status of a subject or sample or can be used as reagents in immunoassays. For instance, the antibodies may be used to detect the presence in a sample of a bacteria having a PS/A antigen on the surface. If the bacteria is present in the sample, then the antibodies may be used to treat the infected subject. The antibodies may also be used to screen bacteria for the presence of PS/A antigen and to isolate PS/A antigen and bacteria containing PS/A antigen from complex mixtures.

The above-described assays and any other assay known in the art can be accomplished by labeling the PS/A or antibodies and/or immobilizing the PS/A or antibodies on an insoluble matrix. The analytical and diagnostic methods for using PS/A and/or its antibodies use at least one of the following reagents: labeled analyte analogue, immobilized analyte analogue, labeled binding partner, immobilized binding partner, and steric conjugates. The label used can be any detectable functionality that does not interfere with the binding of analyte and its binding partner. Numerous labels are known for such use in immunoassays. For example, compounds that may be detected directly, such as fluorochrome, chemiluminescent, and radioactive labels, as well as compounds that can be detected through reaction or derivitization, such as enzymes. Examples of these types of labels include $^{32}P$, $^{14}C$, $^{125}I$, $^{3}H$, and $^{131}I$ radioisotopes, fluorophores such as rare earth chelates or fluorescein and its derivatives, rhodamine and its derivatives, dansyl, umbelliferone, luciferases, such as firefly luciferase and bacterial luciferase (U.S. Patent No. 4,737,456), luciferin, 2,3-dihydrophthalavinediones, horseradish peroxidase (HRP), alkaline phosphatase, β-galactosidase, glucoamylase, lysozyme, saccharide oxidases, such as glucose oxidase, galactose oxidase, and glucose-6-phosphate dehydrogenase. Heterocyclic oxidases such as uricase and xanthine oxidase, coupled to an enzyme that uses hydrogen peroxide to oxidize a dye precursor such as HRP, lactoperoxidase, or microperoxidase, biotin avidin, spin labels, bacteriophage labels, and stable free radicals.

The labels can be conjugated to the PS/A or antibody by methods known to those of ordinary skill in the art. For example, U.S. Patent Nos. 3,940,475 and 3,645,090 demonstrate conjugation of fluorophores and enzymes to antibodies. Other assays which reportedly are commonly used with antigen and antibody and which can be used according to the invention include competition and sandwich assays.

The prior art has described several genes encoding proteins which reportedly lead to the production of a polysaccharide, known as the polysaccharide intracellular adhesin (PI/A). These genes have been cloned and expressed in other species of *staphylococcus*. It has been discovered according to the invention, however, that these genes actually encode the production of proteins which cause the development of PS/A and not PI/A. Thus the invention includes a method of preparing PS/A antigen by producing a PS/A expressing host cell and isolating PS/A antigen.

A PS/A host cell can be prepared by transfecting or transforming a cell with the nucleic acid encoding the *ica* gene (SEQ ID NO: 1). The cell can be a eukaryotic or prokaryotic cell but preferably is a bacterial cell. More preferably the cell is a *staphylococci* which does not ordinarily express PS/A.

The *ica* nucleic acid, in one embodiment, is operably linked to a gene expression sequence which directs the expression of the *ica* nucleic acid within a eukaryotic or prokaryotic cell. The "gene expression sequence" is any regulatory nucleotide sequence, such as a promoter sequence or promoter-enhancer combination, which facilitates the efficient transcription and translation of the *ica* nucleic acid to which it is operably linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, and β-actin. Exemplary viral promoters which function constitutively in cells include, for example, promoters from the simian virus, papilloma virus, adenovirus, human immunodeficiency virus (HIV), Rous sarcoma virus, cytomegalovirus, the long terminal repeats (LTR) of moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively. Such 5' non-transcribing sequences will include a promoter region which includes a promoter sequence for transcriptional control of the operably joined *ica* nucleic acid. The gene expression sequences optionally include enhancer sequences or upstream activator sequences as desired.

The *ica* nucleic acid sequence and the gene expression sequence are said to be "operably linked" when they are covalently linked in such a way as to place the transcription and/or translation of the *ica* coding sequence under the influence or control of the gene expression sequence. If it is desired that the *ica* sequence be translated into a functional protein, two DNA sequences are said to be operably linked if induction of a promoter in the 5' gene expression sequence results in the transcription of the *ica* sequence and if the nature of the linkage between the two DNA sequences does

not (1) result in the introduction of a frame-shift mutation, (2) interfere with the ability of the promoter region to direct the transcription of the *ica* sequence, or (3) interfere with the ability of the corresponding RNA transcript to be translated into a protein. Thus, a gene expression sequence would be operably linked to a *ica* nucleic acid sequence if the gene expression sequence were capable of effecting transcription of that *ica* nucleic acid sequence such that the resulting transcript might be translated into the desired protein or polypeptide.

The *ica* nucleic acid of the invention can be delivered to the host cell alone or in association with a vector. In its broadest sense, a "vector" is any vehicle capable of facilitating: (1) delivery of a nucleic acid molecule containing the genes in the *ica* locus that encode production of proteins that synthesize the PS/A molecule or (2) uptake of a nucleic acid molecule containing the genes in the *ica* locus that encode production of proteins that synthesize the PS/A molecule by a target cell. Preferably, the vectors transport the *ica* molecule into the target cell with reduced degradation relative to the extent of degradation that would result in the absence of the vector. In general, the vectors useful in the invention are divided into two classes: biological vectors and chemical/physical vectors. Biological vectors are useful for delivery/uptake of *ica* nucleic acids to/by a target cell. Chemical/physical vectors are useful for delivery/uptake of *ica* nucleic acids or *ica* polypeptides to/by a target cell.

Biological vectors include, but are not limited to, plasmids, phagemids, viruses, other vehicles derived from viral or bacterial sources that have been manipulated by the insertion or incorporation of the nucleic acid sequences of the invention, and free nucleic acid fragments which can be attached to the nucleic acid sequences of the invention. Viral vectors are a preferred type of biological vector and include, but are not limited to, nucleic acid sequences from the following viruses: retroviruses, such as: Moloney murine leukemia virus; Harvey murine sarcoma virus; murine mammary tumor virus; Rous sarcoma virus; adenovirus; adeno-associated virus; SV40-type viruses; polyoma viruses; Epstein-Barr viruses; papilloma viruses; herpes viruses; vaccinia viruses; polio viruses; and RNA viruses such as any retrovirus. One can readily employ other vectors not named but known in the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. In general, the retroviruses are replication-deficient (i.e., capable of directing synthesis of the desired proteins, but incapable of manufacturing an infectious particle). Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., "Gene Transfer and Expression, A Laboratory Manual," W.H. Freeman Co., New York (1990) and Murry, E.J. Ed. "Methods in Molecular Biology," vol. 7, Humana Press, Inc., Cliffton, New Jersey (1991).

Another preferred virus for certain applications is the adeno-associated virus, a doublestranded DNA virus. The adeno-associated virus can be engineered to be replication -deficient and is capable of infecting a wide range of cell types and species. It further has advantages, such as heat and lipid solvent stability; high transduction frequencies in cells of diverse lineages; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

In addition to the biological vectors, chemical/physical vectors may be used to deliver a *ica* molecule to a target cell and facilitate uptake thereby. As used herein, a "chemical/physical vector" refers to a natural or synthetic molecule, other than those derived from bacteriological or viral sources, capable of delivering the *ica* molecule to a cell.

A preferred chemical/physical vector of the invention is a colloidal dispersion system. Colloidal dispersion systems include lipid-based systems including oil-in-water emulsions, micelles, mixed micelles, and liposomes. A preferred colloidal system of the invention is a liposome. Liposomes are artificial membrane vessels which are useful as a delivery vector in vivo or in vitro. It has been shown that large unilamellar vessels (LUV), which range in size from 0.2 - 4.0 $\mu$m can encapsulate large macromolecules. RNA, DNA, and intact virions can be encapsulated within the aqueous interior and be delivered to cells in a biologically active form (Fraley, et al., Trends Biochem. Sci., (1981) 6:77). In order for a liposome to be an efficient gene transfer vector, one or more of the following characteristics should be present: (1) encapsulation of the gene of interest at high efficiency with retention of biological activity; (2) delivery of the aqueous contents of the vesicle to the target cell cytoplasm at high efficiency; and (3) accurate and effective expression of genetic information.

Liposomes are commercially available from Gibco BRL, for example, as LIPOFECTIN™ and LIPOFECTACE™, which are formed of cationic lipids such as N-[1-(2, 3 dioleyloxy)-propyl]-N, N, N-trimethylammonium chloride (DOTMA) and dimethyl dioctadecylammonium bromide (DDAB). Methods for making liposomes are well known in the art and have been described in many publications. Liposomes also have been reviewed by Gregoriadis, G. in Trends in Biotechnology,

(1985) 3:235-241.

Compaction agents also can be used alone, or in combination with, a biological or chemical/physical vector of the invention. A "compaction agent", as used herein, refers to an agent, such as a histone, that neutralizes the negative charges on the nucleic acid and thereby permits compaction of the nucleic acid into a fine granule. Compaction of the nucleic acid facilitates the uptake of the nucleic acid by the target cell. The compaction agents can be used alone, i.e., to deliver the *ica* molecule in a form that is more efficiently taken up by the cell or, more preferably, in combination with one or more of the above-described vectors.

Other exemplary compositions that can be used to facilitate uptake by a target cell of the *ica* nucleic acids include calcium phosphate and other chemical mediators of intracellular transport, microinjection compositions, electroporation and homologous recombination compositions (e.g., for integrating a *ica* nucleic acid into a preselected location within the target cell chromosome).

**Examples**

**Example 1: Purification of PS/A Antigen.**

It has been discovered according to the invention that PS/A can be purified from any bacterial strain expressing the *ica* locus. Specifically, these include *Staphylococcus epidermidis, Staphylococcus aureus,* and other *Staphylococcal* strains such as *Staphylococcus carnosus* transformed with the genes in the *ica* locus. The following specific strains have been used according to the invention to purify PS/A from include *S. epidermidis* RP62A (ATCC Number: 35984), *S. epidermidis* RP12 (ATCC Number: 35983), *Staphylococcus epidermidis* M187, *S. carnosus* TM300 (pCN27), *S. aureus* RN4220 (pCN27), and *S. aureus* MN8 mucoid.

*1. Method of purification of PS/A from coagulase-negative staphylococci containing the ica locus that encodes production of proteins needed to synthesize PS/A:*

Starting material was prepared from cultures of staphylococci expressing the *ica* genes by growing the bacteria in any growth medium supporting the growth of these staphylococcal strains. A preferred medium is a chemically defined medium (CDM) (Hussain, M., J. G. M. Hastings and P. J. White. 1991. A chemically defined medium for slime production by coagulase-negative Staphylococci. J. Med Microbiol. 34:143) composed of RPMI-1640 AUTO-MOD, an RPMI-1640 preparation modified to allow sterilization by autoclaving; (Sigma Chemical Co., St. Louis, MO) as a starting base. Phenol red was omitted because it readily binds to purified PS/A. The CDM is supplemented with additional amino acids, vitamins, and nucleotides to give it a final composition similar to that described elsewhere (Hussain, M., J. G. M. Hastings and P. J. White. 1991. A chemically defined medium for slime production by coagulase-negative Staphylococci. J. Med. Microbiol. 34:143). The medium was further supplemented with dextrose and sucrose; each was autoclaved separately and then added to a final concentration of 1%.

Cultures were inoculated with a single colony of staphylococci (Muller, E., J. Huebner, N. Gutierrez, S. Takeda, D. A. Goldmann and G. B. Pier. 1993. Isolation and characterization of transposon mutants of Staphylococcus epidermidis deficient in capsular polysaccharide/adhesin and slime. Infect Immun. 61:551) from an agar plate such as trypticase soy agar, that had been incubated overnight at 37°C. Cultures were grown with vigorous mixing at 37°C, with 2 L of $O_2$/min bubbled through via a sparger and the pH maintained at 7.0 by automatic addition of 5 M NaOH with a pH titrator. Cultures were grown until they ceased to need addition of NaOH to maintain the pH at 7.0 (i.e., for 48 - 72 hours).

Crude antigen was extracted from the bacterial cells directly into the culture supernatant with use of divalent cations, low pH, and heat. A preferred method is to add $MgCl_2$ to the culture to a final concentration of 100 mM, and to adjust the pH to 5.0. The culture was then heated and stirred. A temperature of 65°C for 90 min was satisfactory. The cell bodies were sedimented at 9000 X g for 15 min. The supernatant was concentrated to ~1000 ml via tangential-flow filtration (10,000 molecular weight (MW) cutoff filter), and the following buffer exchanges and treatments were performed while the solution was still in the filtration device: The buffer was exchanged with deionized $H_2O$ adjusted to a pH of 5.0 (to remove excesses of $Mg^{2+}$ ions). Next the pH was adjusted to 7.4 with 0.1 M TRIS-0.15 M NaCl, and the solution was treated for 4 hours at room temperature with 5 mg each of RNase and DNase and then for 2 hours with 5 mg of trypsin. The buffer was changed in the tangential flow device to 10 mM EDTA (pH 8.6), and sodium deoxycholate (DOC) added to a final concentration of 3%.

Another preferred method for isolating the polysaccharide antigen of the invention involves incubating the bacteria with a strong base or acid, such as 5 Molar NaOH or HCL. The bacteria are stirred in the strong base or acid for 18-24 hours. The cell bodies are then collected by centrifugation and re-extracted two more times. The supernatant from each of the extractions is pooled and neutrilized to pH 7 using an acid or base. The resulting crude antigen suspension is dialized against deionized water for 2-24 hours. The remaining insoluble crude antigen is collected by centrifugation. The supernatant is tested for the presence of soluble antigen by known assays, such as immunological means. If the

supernatant is positive for soluble antigen, the supernatant can be lyophilized and re-extracted to obtain additional crude antigen. The insoluble crude antigen is resuspended in a buffer solution of 50 mM PBS or 100 mM Tris with 150 mM NaCL.

The suspended crude antigen was treated with 5 mg each of RNase and DNase for 4 hours at 37°C and then with 5 mg of trypsin for 2 hours; the cells were sedimented, and this supernatant added to the concentrated culture supernatant as it was being treated with trypsin. The remaining cell bodies were resuspended in 10 mM EDTA/3% DOC (pH 8.6); stirred for 2 hours and sedimented a third time. The resultant supernatant was added to the concentrated culture supernatant as it was being treated with 10 mM EDTA/3% DOC (pH 8.6). Another alternative is a buffer with a pH>10. A preferred buffer is 0.4 M ammonium carbonate at a pH of 11. Finally, the pooled supernatants were concentrated via stirred-cell filtration using, for example, a 30,000 MW cutoff membrane filter). Particulate or insoluble material was sedimented by centrifugation at 2,000 x g for 30 min and the supernatant retained.

As an alternative to using the above extractions and DOC buffers to obtain dissolved, crude antigen, crude antigen was also obtained from the initial extracts of the cells by adjusting the pH of the extracts to >8.0 with base such as sodium hydroxide, adding 4 volumes of alcohol such as ethanol, recovery of the precipitated material by centrifugation, and redissolution of the precipitate in 0.1 M HCL or any buffer with a pH below 4.0.

High-molecular-weight PS/A-enriched material was next isolated from the crude slime extract by molecular sieve chromatography. A 5.0-cm X 100-cm column was packed with Sepharose CL-4B (Pharmacia, Piscataway, NJ) or a comparable molecular sieve gel and washed with 10 mM EDTA containing 1% DOC (pH 8.6). As an alternative a buffer with a pH < 4.0 was sometimes used. A preferred buffer was 0.1 m glycine-HCL at a pH of 2.0. Crude slime is applied in a volume of 10-20 ml and the PS/A containing fractions were identified by measurement of the absorption of UV light at 206 nm and immuno-dot-blot analysis using a polyclonal rabbit antiserum specific to the PS/A antigen.

Material eluting in the void-volume fractions (MW > 100,000 Daltons) of columns using DOC-based buffers was pooled and treated with proteinase K (0.1 mg/ml), which is proteolytically active in 1% DOC; the material was simultaneously concentrated to 100 ml with a stirred-cell filtration unit (30,000 MW cutoff membrane). Material eluting in the void volume of a molecular sieve column with a low pH buffer (< 4.0) was pooled, sodium phosphate dibasic added to a final concentration of at least 0.1 M and a pH of at least 8.0, and then 4 volumes of alcohol, such as 95% ethanol, was added to precipitate the PS/A antigen.

Further purification of material recovered from DOC columns used a step to destroy remnants of an alkali-labile cell wall antigen present in the preparations. The proteinase K-digested material was treated by the addition of NaOH to a final concentration of 500 mM and the mixture was stirred at 22°C for 2 hours and then neutralized with HCL. PS/A was recovered by alcohol precipitation (such as methanol added to 50% v/v) and sedimented by centrifugation at 30,000 x g for 30 min. Residual DOC and impurities were removed by numerous washes of the precipitate with pure methanol.

To remove residual teichoic acid from material recovered in the void volume of columns run using either the DOC-buffer system or low pH buffer system, the alcohol precipitates were suspended in 24% (v/v) hydrofluoric acid (HF) at 4°C for 48 hours. The HF solution was diluted in water to a concentration of 12% HF, then neutralized with NaOH and dialyzed against running water. PS/A was mostly insoluble at this point. Any remaining soluble PS/A was precipitated by the addition of methanol to 50%, the pellet was washed with pure methanol 3 to 5 times, suspended in deionized $H_2O$, frozen, and lyophilized.

*2. Purification of PS/A from Staphylococcus aureus.*

PS/A was also purified from *S. aureus* using either the above procedure for coagulase-negative staphylococci or a modification of the above procedure. The recovery of PS/A from *S. aureus* preferably was facilitated by using a strain that constitutively makes PS/A such as the MN8 mucoid strain. Such strains can be grown in any medium that supports their growth although a preferred medium is columbia broth. Alternately, any *S. aureus* strain with an intact *ica* locus can be grown in brain heart infusion broth supplemented with glucose at ≥ 0.25% (v/v) to produce PS/A.

Cultures were inoculated with a single colony of *Staphylococcus aureus* from any appropriate medium that had been incubated overnight at 37°C. Cultures were grown with vigorous mixing at 37°C, with 2 L of $O_2$/min bubbled through via a sparger and the pH maintained at 7.0 by automatic addition of 5 M NaOH with a pH titrator. Cultures were routinely grown until they cease to need addition of NaOH to maintain the pH at 7.0 (i.e., for 48 - 72 hours).

The bacterial cells were then recovered from the culture by centrifugation and resuspended in buffered saline. Then lysozyme and lysostaphin enzymes were added in concentrations of >0.1 mg/ml. This suspension was stirred for 2-24 hours at 37 °C, after which a protease was added. A preferred protease was Proteinase K at a concentration of at least 0.1 mg/ml, although other proteases such as Pronase E or trypsin can be used. The mixture was stirred at 37°C for at least 2 hours. The pH of the suspension was then lowered to below 4.0, but preferable to 2.0, using acid such as HCL. The insoluble material was removed by centrifugation and the precipitate re-extracted 3 times by resuspension in low pH (< 4.0, preferably 2.0) solutions followed by stirring for ≥ 10 minutes and centrifugation to remove insoluble materials. The low-pH extracts were pooled, the pH raised to above 8.0 by addition of both sodium phosphate to a concentration of ≥ 0.1 M and a base such as sodium hydroxide, and then 4 volumes of alcohol such as 95% ethanol

were added. Another alternative is a buffer with a pH>10. A preferred buffer is 0.4 M ammonium carbonate at a pH of 11.

Another preferred method for isolating the polysaccharide antigen of the invention involves incubating the bacteria with a strong base or acid, such as 5 M NaOH or HCL. The bacteria are stirred in the strong base or acid for 18-24 hours. The cell bodies are then collected by centrifugation and re-extracted two more times. The supernatant from each of the extractions is pooled and neutralized to pH 7 using an acid or base. The resulting crude antigen suspension is dialyzed against deionized water for 2-24 hours. The remaining insoluble crude antigen is collected by centrifugation. The supernatant is tested for the presence of soluble antigen by known assays, such as immunological means. If the supernatant is positive for soluble antigen, the supernatant can be lyophilized and re-extracted to obtain additional crude antigen. The insoluble crude antigen is resuspended in a buffer solution of 50 mM PBS or 100 mM Tris with 150 mM NaCL. The material was then subjected to the same enzymatic treatments as described above with respect to purification method of PS/A from coagulase-negative *staphylococci.*

The insoluble material was then recovered by centrifugation, redissolved in low pH (<4.0, preferably 1.0) solutions, insoluble material was removed by centrifugation, and the high molecular weight material in the soluble fraction recovered by application to a molecular sieve column equilibrated in a buffer below pH 4.0. The preferred buffer was 0.1 M glycine-HCL at a pH of 2.0. A typical molecular sieve column has dimensions of 2.6 X 100 cm and was packed with a gel such as Sephacryl S-500 (Pharmacia). The PS/A containing fractions were identified by measurement of the absorption at 206 nm and immuno-dot-blot analysis with polyclonal rabbit antiserum to PS/A. Immunologically reactive fractions eluting in the high molecular (> 100,000) weight range were pooled, the pH raised to >8 by addition of both sodium phosphate to a concentration of ≥ 0.1 M and a base (such as sodium hydroxide) followed by addition of 4 volumes of an alcohol such as ethanol and the resultant precipitate recovered by centrifugation. The alcohol precipitates were next suspended in 24% (v/v) hydrofluoric acid (HF) at 4°C for 48 hours. The HF solution was diluted in water to a concentration of 12% HF, then neutralized with NaOH and dialyzed against running water. PS/A was mostly insoluble at this point. Any remaining soluble PS/A was precipitated by the addition of alcohol to >50% final concentration, the pellet was washed with alcohol such as 95% ethanol or pure methanol 3 to 5 times, and the pellet was suspended in deionized $H_2O$, frozen, and lyophilized.

**Example 2: Chemical Synthesis of PS/A antigen.**

***Methods:***

PS/A is chemically similar to chitin (poly-N-acetyl-glucosamine with beta-1-4 linkages) and chitosan (poly-glucosamine with beta-1-4 linkages). The N-acetyl groups of chitin were readily removed by treatment with mild base (1.0 M NaOH, 95°C, 1 hour)--to yield chitosan. The free amino group was then substituted with succinate using succinic acid added to these materials in the presence of a water soluble carbodiimide reagent that promotes the coupling of free amino groups to free carboxyl groups.

10 mg of chitosan was suspended in 1.0 of 0.1 M HC1 and stirred until a gel-like suspension was obtained. One-hundred mg of succinic acid was then added followed by 75 mg of 1-ethyl-3-(3-dimethylaminopropyl) carbodiimide -HCl (EDC, Sigma) and the suspension stirred for 2 days at room temperature. The solution was then neutralized with 2 M sodium hydroxide, u bound succinic acid and free EDC removed by dialysis against water, and the succinylated chitosan lyophilized.

***Results:***

The resulting material was similar to the native poly-N-succinyl glucosamine of PS/A, differing by the nature of the linkages between the individual glucosamine residues. However, we have found according to the invention that antibodies raised to PS/A will react with N-succinylated chitosan, indicating that proper chemical modification of chitin and chitosan results in a molecule with the ability to elicit antibodies to PS/A.

**Example 3: Analysis of the PS/A antigen.**

Use of both base (NaOH) and hydrofluoric acid treatments to degrade contaminants significantly improve the purity of PS/A over prior art preparations previously obtained. PS/A eluted in the high molecular weight, void-volume fractions of a Sepharose 4B column (>100,000 kDa) and gave a single precipitin band in immunodiffusion, as previously shown (Tojo, M., N. Yamashita, D. A. Goldmann and G. B. Pier. 1988. Isolation and characterization of a capsular polysaccharide/adhesin from Staphylococcus epidermidis. J Infect Dis. 157: 713). In addition, sensitization of ELISA wells with dilutions of PS/A down to 0.02 μg/well resulted in the binding of significantly more of a 1:500 dilution of antibody to purified PS/A than of preimmune serum. Use of 3% DOC or low pH buffers (< 4.0) to solubilize PS/A during purification was found to be important, but once the PS/A was precipitated from these solutions by methanol, it could not be resolubilized at a pH of> 4.0, even in DOC buffers. PS/A was slightly soluble (~500 μg/ml) in 50% propanol-50% butanol and in pyridine. The PS/A antigen was devoid of detectable phosphate (<.01%) (Keleti, G. and W.H. Lederer. 1974. Handbook of Micromethods for the Biological Sciences. Van Nostrand Reinhold Co., New York).

**Example 4: Anaylsis of the Chemical Properties of PS/A.**

When we analyzed PS/A isolated from *S. epidermidis* M187, *S. carnosus* (pCN27) and *S. aureus* MN8 mucoid after hydrolysis in 4 M HCL acid at high temperature (95-100 °C) for 14 hours, we found that glucosamine was the single sugar component in PS/A using combinations of gas-liquid chromatography-mass spectrometry (GLC-MS) and nuclear magnetic resonance (NMR). A representative NMR spectrum is shown in Figure 1. In addition, a high quantity of succinate (about equimolar with glucosamine) was also identified in the preparations. The succinate was released from the antigens by hydrolysis in 5 M NaOH for ≥10 min at 95 °C, but not with 1 M NaOH under the same conditions. The lower amount of NaOH would readily release carboxyl-ester-linked succinate. Along with NMR results, this establishes the succinate as linked to the amino, and not hydroxyl, groups of glucosamine. Hydrolysis of PS/A into oligosaccharide fragments by ozone and analysis by NMR established the linkage between the sugars as a beta linkage. PS/A isolated from these strains loses serologic reactivity and chemical properties detectable by GLC-MS after treatment with sodium periodate (0.2 M for 14 h), indicating a 1-6 linkage between the glucosamine residues. Also, hydrolysis with 5 M NaOH for >5 min destroys serologic activity.

Thus the native PS/A material is a high molecular weight (>100,000 Daltons) homopolymer of N-succinyl D-glucosamine residues linked to each other in a beta 1-6 linkage. The level of substitution of the succinate groups on the amino group of the glucosamine approaches 100% in the native molecule.

**Example 5: Immunization with purified PS/A elicits antibodies reactive with the antigen.**

**Method:** Previously described methods for eliciting antibodies to PS/A by immunization of rabbits (Kojima, Y., M. Tojo, D. A. Goldmann, T. D. Tosteson and G. B. Pier. 1990. Antibody to the capsular polysaccharide/adhesin protects rabbits against catheter related bacteremia due to coagulase-negative staphylococci. J Infect Dis. 162:435*;* Tojo, M., N. Yamashita, D. A. Goldmann and G. B. Pier. 1988. Isolation and characterization of a capsular polysaccharide/adhesin from Staphylococcus epidermidis. J Infect Dis. 157: 713*;* Takeda, S., G. B. Pier, Y. Kojima, M. Tojo, E. Muller, T. Tosteson and D. A. Goldmann. 1991. Protection against endocarditis due to Staphylococcus epidermidis by immunization with capsular polysaccharide/adhesin. Circulation. 84:2539*)* were used to prepare antisera from the antigen isolated from *S. epidermidis* M187 and *S. aureus* MN8 mucoid.

**Results:** The antibodies elicited by preparations from both bacterial strains induced antibodies of comparable titers to both antigens (Table 1).

**TABLE I**

| Binding of antibodies raised to MN8-mucoid and S. epidermidis PS/A to each others antigen in ELISA | |
|---|---|
| **ELISA for MN8-mucoid coated PS/A plates (2μg/well)** | |
| **Anti-sera diluted 1:500** | **O.D $_{405\ nm}$** |
| **anti-MN8 mucoid PS/A** | **0.856** |
| **anti-S. epidermidis PS/A** | **0.872** |
| **Normal rabbit sera** | **0.000** |
| **ELISA for S. epidermidis coated PS/A plates (2pg/well)** | |
| **Anti-sera diluted 1:500** | **O.D $_{4050m}$** |
| **anti-MN8 mucoid PS/A** | **0.770** |
| **anti-S. epidermidis PS/A** | **0.886** |
| **Normal rabbit sera** | **0.013** |

**Example 6: Physical location of PS/A antigen on staphylococcal strains.**

Methods: *S. aureus* strains MN8 mucoid and RN4220 (pCN27) were grown on trypticase soy agar, and *S. aureus* MN8 was grown in Brain Heart Infusion Broth supplemented with ≥0.25% glucose. The cells were then probed

first with polyclonal rabbit antiserum to PS/A and then with gold-labeled protein A, or gold-labeled anti-rabbit IgG, and micrographs were obtained.

PS/A has been previously shown to represent the bacterial capsule for *S. epidermidis.* We investigated whether strains carrying the *ica* locus were encapsulated using immunoelectron microscopy. A series of immunoelectron microscopic photographs of: (a) interaction of anti-PS/A antibodies with a PS/A capsule on *S. aureus* strain MN8 mucoid; (b) negative control using normal rabbit serum (NRS) staining of *S. aureus* strain MN8 mucoid; (c) interaction of anti-PS/A antibodies with PS/A capsule on *S. aureus* strain RN4220(pCN27); (d) negative control using NRS staining of *S. aureus* strain RN4220; (e) interaction of anti-PS/A antibodies with *S. aureus* strain MN8 grown in brain heart infusion broth supplemented with 0.25% glucose; and (f) negative control using NRS staining of *S. aureus* strain MN8 grown in brain heart infusion broth supplemented with 0.25% glucose were taken. The micrographs demonstrate that antibodies to PS/A bind to an extracellular capsule in these strains.

**Example 7: Expression of PS/A antigen by human clinical isolates of *S. aureus.***

Many human clinical isolates of *S. epidermidis* express PS/A antigens constitutively (i.e., all of the time under a variety of growth conditions). Also, some strains of *S. aureus* do the same (i.e., MN8 mucoid). However most human clinical isolates do not express much PS/A when grown under normal laboratory conditions. We found that if these *S. aureus* strains were grown in brain heart infusion broth medium containing ≥0.25% glucose, then expression of PS/A could be detected by serologic means such as an ELISA inhibition assay (shown in Figure 2). In addition, we found that isolates of *S aureus* that do not express detectable PS/A prior to infection of mice can be shown to produce PS/A when recovered from an infected tissue such as the kidney. Thus under specialized *in vitro* conditions or following experimental infections in animals, strains of *S. aureus* that otherwise do not make detectable PS/A now do so. Another medium, we have found to enhance PS/A expression *in vitro* is Staphylococcus 110 medium.

An immunoelectron microscopy photograph of a human clinical isolate (ASEAN) freshly isolated from agar to detect PS/A expression and stained with (a) anti-PS/A antibodies, or (b) NRS control was taken. We found that human clinical isolates of *S. aureus* express PS/A when strains were tested by transmission electron microscopy from an agar plate that was first used to detect infection in a sample of fluid or tissue. Thus if human clinical isolates were tested for PS/A expression without extensive passage of the bacteria on laboratory media then it can be shown that they make PS/A antigen.

**Example 8: Expression of PS/A antigen by animal isolates of *S. aureus.***

*S. aureus* is the major cause of mastitis infections in animals and causes considerable economic losses. We have also analyzed *S. aureus* isolates from animals (cows and sheep) for the expression of PS/A. We have found many of the isolates analyzed make the PS/A antigen, as determined by serologic testing. Out of 40 animal isolates tested, 23 had strong reactions in immuno-dot blots with antibodies to PS/A. This was detectable even though the isolates were not grown in special media to enhance production of PS/A as must be done with human isolates. Thus, unlike human clinical isolates of *S. aureus,* many animal isolates seem to be capable of making the PS/A constitutively.

**Example 9: Protection against *S. aureus* infection by antibodies** to PS/A.

***Methods:*** PS/A antigen 1-100 μg (preferred 100 μg/mouse) was used to immunize mice to elicit antibodies. The mice were then challenged with live *S. aureus* bacteria, either *S. aureus* strain Reynolds or strain MN8 and those with antibodies to PS/A were shown to be protected against infection.

***Results:*** Actively immunizing mice with PS/A followed by intravenous challenge with S. *aureus* reduced the number of bacteria in the kidney's five days later when the animals were killed and tested (Figure 3).

Similarly, giving mice antibodies raised in rabbits to the PS/A antigen followed by challenge of the mice with *S. aureus* strains protects the mice against *S. aureus* infection (Figure 4). Thus antibodies to PS/A protect mice against *S. aureus* infection. As noted above, analysis of the isolates recovered from the infected kidneys of nonimmune control mice showed that infection had induced expression of PS/A during the time of infection in the animal.

**Example 10: Detection of genes for PS/A production in isolates of *S. aureus.***

***Methods: DNA extraction from bacteria, PCR analysis and Souther blot analysis*:**

DNA preparation: Bacteria were inoculated in 10-2- ml of trypticase soy broth (TXB) and grown overnight at 37°C. The resulting growth was recovered by centrifugation. The bacterial cells were resuspended in lysis buffer (25mM Tris, pH 8.0, 25 mM EDTA, 300 mM sucrose). Cells were then incubated with lysostaphin (0.2 mg/ml) and lysozyme (2

mg/ml) for 1-3 hours at 37°C. Following this enzyme digestion, 0.5 ml of 5% sodium dodecyl sulfate (SDS) in 45% etanol was added, vortexed and incubated for 30 min at room temperature. One ml of buffered phenol/chloroform/isoamyl alcholo (volume ratios of 27:12:1) was then added followed by vortexing and centrifugation (15 min, 5000 rpm, 4°C). The upper aqueous layer was removed and added to chloroform:Isoamyl alchol (24:1 ratio). Again this was centrifuged (15 min, 5000 rpm, 4°C). the DNA was recovered in the upper aqueous layer, then precipitated by addition of 95% ethanol to amount equal to double the starting volume, and the precipitated DNA spooled onto a glass rod. The DNA was then dissolved in distilled water.

Restriction enzyme digestion of NDA for Southern blot analysis was done according to the manufacturer's protocol (Gibco BRL, Grand Island, NY). The DNA probe was labeled using the ECL protocol (Amersham International plc, Buckinghamshire, England) following the manufacturer's instructions.

PCR was performed using a touch down protocol. Briefly, primers (SEQ. ID. NO. 2-3) were added to the DNA obtained from the Staphylococcal strains (200nM final concentration) and the thermal cycler programmed to perform DNA melting at 95 °C and polymerase extensions starting at 60°C with decreasing annealing temperatures of 0.5°C used at each cycle until reaching 45 °C.

Results: Using the polymerase chain reaction (PCR) we have shown that isolates of *S. aureus* have the genes to make PS/A. These genes are contained in a locus designated *ica* and were originally isolated and sequenced by Heilmann et al. (Heilmann, C., O. Schweitzer, C. Gerke, N. Vanittanakom, D. Mack and F. Gotz. 1996. Molecular basis of intercellular adhesion in the biofilm-forming Staphylococcus epidermidis. Molec. Microbiol. 20:1083). We demonstrate herein that the *ica* locus encodes proteins that synthesize the poly-N-succinyl glucosamine molecule PS/A and not the poly-N-acetylated polyglucosamine molecule termed PIA.

Primers were designed for the PCR analysis based on the sequence of the *ica* genes to amplify a fragment of DNA of 2.7 kB (SEQ ID No: 2 and 3). When these primers were mixed with DNA isolated from *S. aureus* strains MN8, MN8 mucoid, RN450, Becker, Reynolds and 1A , along with DNA from *S. epidermidis* strain RP62A, from which the *ica* genes had originally been cloned, and used in a PCR reaction we found that all of the *S. aureus* strains had the *ica* locus (Figure 5). When the amplified 2.7 kB fragment was labeled and used as a probe in a Southern blot reaction of DNA extracted from these *S. aureus* strains, we also detected the presence of the genes in the *ica* locus (Figure 6). We have thus shown that the genes needed to make to proteins to synthesize the PS/A antigen are present in *S. aureus.*

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.

All references, patents and patent publications that are recited in this application are incorporated in their entirety herein by reference. We claim:

SEQUENCE LISTING

<110> The Brigham and Women's Hospital, Inc.
Pier, Gerald B.
McKenney, David
Wang, Ying

<120> CAPSULAR POLYSACCHARIDE ADHESIN ANTIGEN PREPARATION, PURIFICATION AND USE

<130> B0801/7144/HCL

<150> US 60/093,117
<151> 1998-07-15

<160> 3

<170> FastSEQ for Windows Version 3.0

<210> 1
<211> 4500
<212> DNA
<213> Staphylococcus Epidermidis

<400> 1
tttgaaatct cgaatttgtt acatactagt tacaaaaatt attttttaa aaatacattt 60
aacagtgaat atacttggtc tttaaaacgg tttttactgt cctcaataat cccgaatttt 120
tgtgaaaagg aggctctaaa ataccaagtc tcaagaaaaa gaagaattaa gtttataaag 180
tcctctttat ccaaagcgat gtgcgtagga tcataatact ttatcaattc atcatgtaag 240
gtagtattaa tttcttgaag atggtgtttg atttctgaat tcagtgcttc tggagcacta 300
gataattgaa catataattt aatatatctc tcatcaacgt cgaatataaa tttgaataaa 360
aactggtaaa gtccgtcaat ggaataatta tcatcatggt tcctaagcaa aaaatctata 420
aagtaattga aacaattctc aacacttttt cgatatattt cttccttatt atcgtaatga 480
taatatagac tagccttttt tatatttaca cttttagaaa tatcatcaag tgtagtacca 540
tcgtacccct tttcggaaaa taaggttatt gcgttatcaa taatcttatc tttcaattct 600
aaaatctccc ccttattcaa ttttctaaaa atatattaca gaaaaattaa gttaaaatta 660
caaatattac tgtttcagta taacaacatt ctattgcaaa ttgaaatact ttcgattagc 720
atatgcttta caacctaact aacgaaaggt aggtgaaaaa atgcatgtat ttaacttttt 780
acttttctat ccaattttta tgtcaattta ctggatagta ggatcgattt actatttttt 840
tattaaagaa aaacccttta atcgatcatt gttagtaaaa tctgaacatc aacaagttga 900
aggcatctcc tttttattag cttgctacaa tgaaagtgaa acagttcaag acacgctttc 960
tagtgtttta tctctagaat atcctgaaaa agaaattatc attatcaatg atggaagttc 1020
tgataatact gctgaaatca tctatgactt caagaaaaat catgatttta aatttgttga 1080
cctcgaagtc aatagaggta aagctaatgc actcaatgag ggaatcaaac aagcatctta 1140
cgaatatgtt atgtgtttag atgctgacac tgtcattgat gacgatgcgc ctttttatat 1200
gattgaagac tttaaaaaga atccaaaatt aggcgcagtt acaggtaatc cacgtattcg 1260
taataaaagt tctattttag gaaaaataca gaccattgaa tatgcaagta ttattggttg 1320
tatcaagcga agtcaatctc ttgcaggagc aatcaatact atttcaggtg ttttcacact 1380
atttaaaaaa agtgcactca aagatgtagg ttattgggat actgacatga ttactgagga 1440
tattgctgtt tcatggaaac tccatctttt tgattacgaa attaagtacg aaccacgtgc 1500
tctatgctgg atgttagtgc ctgaaactat aggtggttta tggaaacaaa gggttcgatg 1560
ggctcaaggc gggcatgaag tacttttaag agacttttgg ccaacaatta aaactaagaa 1620
attatcacta tatattttaa tgtttgaaca aatcgcatcg attacatggg tctacatcgt 1680

actatgttat ttatcttttt tagtaatcac agccaacatc ttagattaca catatttaaa 1740
atatagtttt tcaatctttt tcttttcatc ctttacgatg acctttatca atatcatcca 1800
atttacagtt gccttattta ttgacagtcg ctacgaaaag aaaaatatag ttggcctgat 1860
atttttaagt tggtatccaa cgttatactg ggttatcaat gccgcagttg tcattatggc 1920
atttcctaaa gcattaaaaa gaaagaaagg tggctatgct acatggtcaa gcccagacag 1980
aggcaatatc caacggtaac ctcttattta aatatagtta gggagagctt atttattact 2040
atatccggag tattttggat gtattgtatc gttgtgatga ttgtttatat aggaactctt 2100
atcaattctc aaatggaaag tgttataaca atacgtattg cattaaatgt tgaaaacacg 2160
gaaatttaca aattattcgg atggatgagt ttgtttgtac ttattatatt tatctttttt 2220
acatttagtc tcgcgtttca aaaatataag aaaggtcgtg acatatgaaa cctttcaaat 2280
taatctttat tagcgcattg atgatattaa taatgacgaa tgcaacacca atatcacacc 2340
tgaatgctca agctaatgaa gaaaacaaga agttaaagta cgaaaaaaat agcgcactcg 2400
cgttaaacta tcacagagta agaaaaaagg atcctttgaa tgactttata tcattactat 2460
ctgggagtaa ggaaattaaa aattatagtg tcactgatca agaatttaaa tcacaaattc 2520
aatggcttaa agcacacgac gcaaagtttt taactttgaa agaatttatt aaatataaag 2580
aaaaaggtaa atttcctaaa agaagtgttt ggattaactt tgatgatatg gatcaaacga 2640
tttatgacaa tgcctttcct gttttgaaaa aatatcatat tccagcaaca ggttttctta 2700
ttacgaacca cattggttct accaattttc ataatttaaa tttactttca aaaaagcaat 2760
tagatgaaat gtatgaaaca ggcttatggg actttgaatc tcatactcat gatttacacg 2820
ctcttaagaa aggcaataaa tcgaagtttt tagattcgtc tcaatctgtt gctagtaaag 2880
atattaaaaa aagcgaacac tatttaaata aaaactaccc aaaaaatgaa cgcgcacttg 2940
cttacccata cggattaatt aatgacgaca aaataaaagc tatgaaaaaa aatggaattc 3000
aatatgggtt tacacttcag gaaaaagctg tcacaccaga tgccgataac tatagaattc 3060
cacgtatttt agtaagtaat gatgcatttg aaacgctaat aaaggaatgg gacggattcg 3120
atgaagaaaa ataaacttga attagtgtat ttacgtgcgt ttatttgtgt cataatcatc 3180
gtgacacact tactaacgca aatcacttta gaaaatgaac agatgtctga tagttcactc 3240
atattgcaat attatatacg caatattttt attttcggca cccctagttt tataatattg 3300
tctcaattat taacaacatt aaattacgaa tcagtaacta taaattatct tttttcaaga 3360
tttaagtata tttttattcc atatctttta atcggcttgt tctatagtta tagtgaatca 3420
cttatcaccg cttcttcttt taaaaagcag tttatcgaaa atgttgtttt aggacaatgg 3480
tatggctatt tcattatcat aattatgcag ttctttgttc tatcttatat catttacaaa 3540
attaatttta gattgttcaa tagtaaaatt ttgctgcttt tagcatttat agtccaacaa 3600
tcttatctac attatttttt gaataatgac acttttcatc aattcatgac tcattattat 3660
ccattaagcg aaaatacaat gatattagga tggatattct actttttctt aggtggttac 3720
attggctata attatgaaaa aatattatct ttcttagaaa aatatttaat tatagttatt 3780
atgttaactt taggcgcata tgttttattt atcgctgttt ccggtagtga ttattggaat 3840
gtcacaagct ttacttatac gttaacatta tataatagtg tcatgttctt cttattacta 3900
ggagtctgta tgcactttaa aactatgtta ttaaatacta ttaaagctat tagtgcattt 3960
tcctttttca tttatttgtt gcacccaatt atcttagatt ctctttttgc ttacaccaac 4020
atatttgaag ataatacaat tgttttcttg gcgatttcac ttttaatgat tctaggaatt 4080
tgtataggcg tcggaatgat gttaagagag ttttatatat tcagatttgt aattggaaaa 4140
caaccgtaca agttacaatt tgaccaatac cagcctaact ggaattaata aaaaaagtcc 4200
cttattcaag ctatggctta tgtatgtgct tgaataaggg attttatctt actatagttt 4260
cacattatga aaataaattt tttaatattc tgtataaaga gcctaataat tgaaagaaat 4320
cacctgtcat gtatctcact cctatctata taagattacg ttaggtttat accctatatc 4380
attttattta tttgttgatg ttaattgttc actttgtact aaatcatcag caagaccatg 4440
ccaaaactgt tgcaattcat ctctagtacg ttttgtatct gtactgtctt gtcctacaaa 4500

<210> 2
<211> 20
<212> DNA
<213> Artificial Sequence

<220>

<223> Primer

<400> 2
tgcactcaat gagggaatca 20

<210> 3
<211> 21
<212> DNA
<213> Artificial Sequence

<220>
<223> Primer

<400> 3
aatcactacc ggaaaacagc g 21

## Claims

1. A composition comprising:

a pure capsular polysaccharide/adhesin having the following structure:

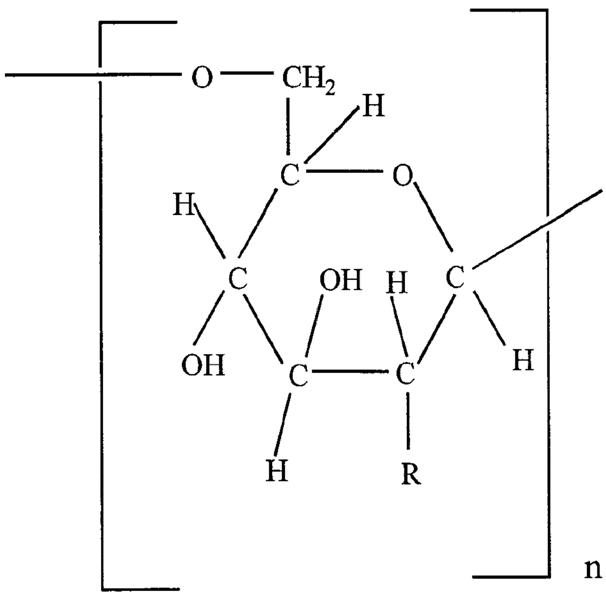

wherein n is an integer greater than or equal to 3, wherein R is selected from the group consisting of $-NH-CO-(CH_2)_m-COOH$, $-NH-(CH_2)_m-(COOH)_2$, $-NH-(CH_2)_m-COOH$, and $-NH_2$, wherein m is 2-5, and wherein at least 10% of the R groups are selected from the group consisting of $-NH-CO-(CH_2)_m-COOH$, $-NH-(CH_2)_m-(COOH)_2$, and $-NH-(CH_2)_m-COOH$.

2. The composition of claim 1, wherein at least 50% of the R groups are selected from the group consisting of $-NH-CO-(CH_2)_m-COOH$, $-NH-(CH_2)_m-(COOH)_2$, and $-NH-(CH_2)_m-COOH$.

3. The composition of claim 1, wherein at least 75% of the R groups are selected from the group consisting of -NH-

$CO-(CH_2)_m-COOH$, $-NH-(CH_2)_m-(COOH_2)$, and $-NH-(CH_2)_m-COOH$.

4. The composition of claim 1, wherein at least 90% of the R groups are selected from the group consisting of $-NH-CO-(CH_2)_m-COOH$, $-NH-(CH_2)_m-(COOH)_2$, and $-NH-(CH_2)_m-COOH$.

5. The composition of claim 1, wherein at least 10% of the R groups are $-NH-CO-CH_2-CH_2-COOH$.

6. The composition of claim 1, wherein at least 50% of the R groups are $-NH-CO-CH_2-CH_2-COOH$.

7. The composition of claim 1, wherein at least 90% of the R groups are $-NH-CO-CH_2-CH_2-COOH$.

8. The composition of claim 1, wherein the capsular polysaccharide/adhesin has a molecular weight of greater than 25,000.

9. The composition of claim 1, wherein the capsular polysaccharide/adhesin has a molecular weight of greater than 30,000.

10. The composition of claim 1, wherein the capsular polysaccharide/adhesin has a molecular weight of greater than 100,000.

11. The composition of claim 1, wherein the capsular polysaccharide/adhesin is a homo-substituted polymer.

12. The composition of claim 1, wherein the capsular polysaccharide/adhesin is a hetero-substituted polymer.

13. The composition of claim 1, wherein R is selected from the group consisting of$-NH-CO-CH_2-CH_2-CH_2-COOH$, $-NH-CO-CH_2CH_2-CH_2-CH_2-COOH$, and $-NH-CO-CH_2-CH_2-CH_2-CH_2-CH_2-COOH$.

14. The composition of claim 1, wherein R is selected from the group consisting of $-NH-CH_2-CH_2-COOH$,- $NH-CH_2-CH_2-CH_2-COOH$, $-NH-CH_2-CH_2-CH_2-CH_2-COOH$, and $-NH-CH_2-CH_2-CH_2-CH_2-CH_2-COOH$.

15. The composition of claim 1, wherein R is selected from the group consisting of $-NH-CH_2-CH_2-(COOH)_2$,$-NH-CH_2-CH_2-CH_2-(COOH)_2$,$-NH-CH_2-CH_2-CH_2-CH_2-(COOH_2)$, and $-NH-CH_2-CH_2-CH_2-CH_2-CH_2-(COOH)_2$.

16. The composition of claim 1, wherein the capsular polysaccharide/adhesin is formulated as a vaccine.

17. The composition of claim 1, further comprising a carrier compound conjugated to the capsular polysaccharide/ adhesin.

18. The composition of claim 17, wherein the carrier compound is conjugated to the capsular polysaccharide/adhesin through a linker.

19. The composition of claim 1, wherein the capsular polysaccharide/adhesin is at least 95% pure.

20. The composition of claim 1, wherein the capsular polysaccharide/adhesin is at least 97% pure.

21. The composition of claim 1, wherein the capsular polysaccharide/adhesin is at least 99% pure.

22. A composition, comprising:

a capsular polysaccharide/adhesin having the following structure:

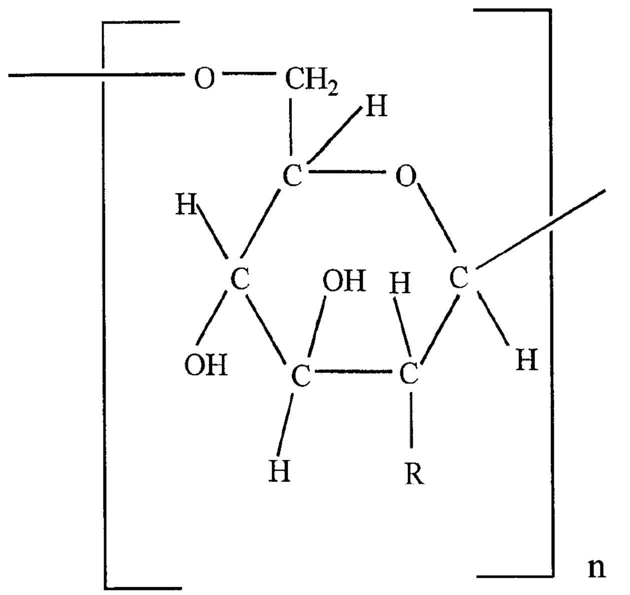

wherein n is an integer greater than or equal to 3, wherein R is selected from the group consisting of -NH-CO-$(CH_2)_{3-5}$-COOH, -NH-$(CH_2)_m$-$(COOH)_2$, -NH-$(CH_2)_m$-COOH, and -$NH_2$ wherein m is 2-5, and wherein at least 10% of the R groups are selected from the group consisting of -NH-CO-$(CH_2)_{3-5}$-COOH, -NH-$(CH_2)_m$-$(COOH)_2$, and -NH-$(CH_2)_m$-COOH.

**23.** The composition of claim 22, wherein at least 50% of the R groups are selected from the group consisting of -NH-CO-$(CH_2)_{3-5}$-COOH, -NH-$(CH_2)_m$-$(COOH)_2$, and -NH-$(CH_2)_m$-COOH.

**24.** The composition of claim 22, wherein at least 75% of the R groups are selected from the group consisting of -NH-CO-$(CH_2)_{3-5}$-COOH, -NH-$(CH_2)_m$-$(COOH)_2$, and -NH-$(CH_2)_m$-COOH.

**25.** The composition of claim 22, wherein at least 90% of the R groups are selected from the group consisting of -NH-CO-$(CH_2)_{3-5}$-COOH, -NH-$(CH_2)_m$-$(COOH)_2$, and -NH-$(CH_2)_m$-COOH.

**26.** The composition of claim 22, wherein the capsular polysaccharide/adhesin has a molecular weight of greater than 25,000.

**27.** The composition of claim 22, wherein the capsular polysaccharide/adhesin has a molecular weight of greater than 30,000.

**28.** The composition of claim 22, wherein the capsular polysaccharide/adhesin has a molecular weight of greater than 100,000.

**29.** The composition of claim 22, wherein the capsular polysaccharide/adhesin is a homo-substituted polymer.

**30.** The composition of claim 22, wherein the capsular polysaccharide/adhesin is a hetero-substituted polymer.

**31.** The composition of claim 22, wherein the capsular polysaccharide/adhesin is formulated as a vaccine.

**32.** The composition of claim 22, further comprising a carrier compound conjugated to the capsular polysaccharide/adhesin.

**33.** The composition of claim 32, wherein the carrier compound is conjugated to the capsular polysaccharide/adhesin through a linker.

**34.** A composition, comprising:

a capsular polysaccharide/adhesin having the following structure:

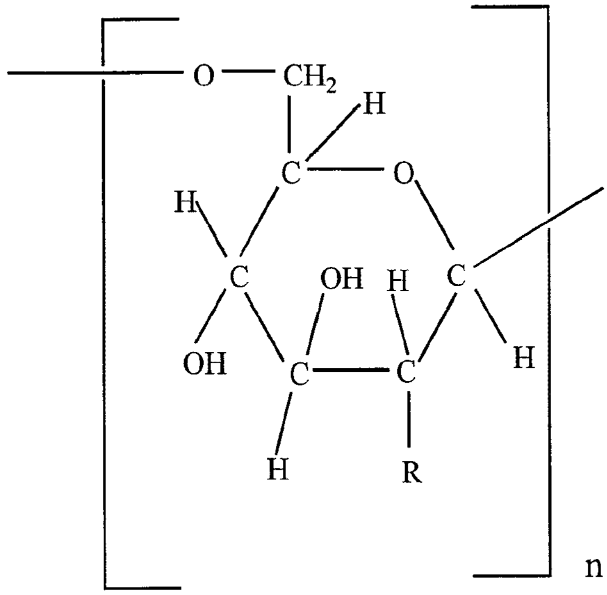

wherein n is an integer greater than or equal to 3, wherein R is selected from the group consisting of -NH-CO-$CH_2$-$CH_2$-COOH and -$NH_2$ and wherein between 10 and 90% of the R groups are -NH-CO-$CH_2$-$CH_2$-COOH.

**35.** The composition of claim 34, wherein between 20 and 80% of the R groups are -NH-CO-$CH_2$-$CH_2$-COOH.

**36.** The composition of claim 34, wherein between 30 and 70% of the R groups are -NH-CO-$CH_2$-$CH_2$-COOH.

**37.** The composition of claim 34, wherein between 40 and 60% of the R groups are -NH-CO-$CH_2$-$CH_2$-COOH.

**38.** The composition of claim 34, wherein the capsular polysaccharide/adhesin has a molecular weight of greater than 25,000.

**39.** The composition of claim 34, wherein the capsular polysaccharide/adhesin has a molecular weight of greater than 30,000.

**40.** The composition of claim 34, wherein the capsular polysaccharide/adhesin has a molecular weight of greater than 100,000.

**41.** The composition of claim 34, wherein the capsular polysaccharide/adhesin is a homo-substituted polymer.

**42.** The composition of claim 34, wherein the capsular polysaccharide/adhesin is a hetero-substituted polymer.

**43.** The composition of claim 34, wherein the capsular polysaccharide/adhesin is formulated as a vaccine.

**44.** The composition of claim 34, further comprising a carrier compound conjugated to the capsular polysaccharide/adhesin.

**45.** The composition of claim 44, wherein the carrier compound is conjugated to the capsular polysaccharide/adhesin through a linker.

**46.** A composition, comprising:

a capsular polysaccharide/adhesin composed of repeating monomer units wherein the monomer unit is selected from the group consisting of α-1-6 polyglucose, α-1-4 polyglucose, α-1-3 polyglucose, β-1-4 polyglucose, β-1-3

polyglucose, and α-1-4 polygalactose and wherein C2 of the monomer unit is substituted with an R group selected from the group consisting of-NH-CO-$(CH_2)_m$-COOH, -NH-$(CH_2)_m$-$(COOH)_2$, -NH-$(CH_2)_m$-COOH, and -$NH_2$, wherein m is 2-5, and wherein at least 10% of the R groups are selected from the group consisting of-NH-CO-(CH2)m-COOH, -NH-$(CH_2)_m$-$(COOH)_2$, and -NH-$(CH_2)_m$-COOH.

**47.** The composition of claim 46, wherein the monomer unit is selected from the group consisting of α-1-6 polyglucose, α-1-4 polyglucose, α-1-3 polyglucose, β-1-4 polyglucose, and β-1-3 polyglucose.

**48.** The composition of claim 47, wherein the polyglucose backbone is in an α-1-6 formation.

**49.** The composition of claim 47, wherein the polyglucose backbone is in a β-1-4 formation.

**50.** The composition of claim 47, wherein the polyglucose backbone is in a β-1-3 formation.

**51.** The composition of claim 47, wherein at least 50% of the R groups are selected from the group consisting of -NH-CO-$(CH_2)_m$-COOH, -NH-$(CH_2)_m$-$(COOH)_2$, and -NH-$(CH_2)_m$-COOH.

**52.** The composition of claim 47, wherein at least 75% of the R groups are selected from the group consisting of -NH-CO-$(CH_2)_m$-COOH, -NH-$(CH_2)_m$-$(COOH)_2$, and -NH-$(CH_2)_m$-COOH.

**53.** The composition of claim 47, wherein at least 90% of the R groups are selected from the group consisting of -NH-CO-$(CH_2)_m$-COOH, -NH-$(CH_2)_m$-$(COOH)_2$, and -NH-$(CH_2)_m$-COOH.

**54.** The composition of claim 47, wherein at least 10% of the R groups are -NH-CO-$CH_2$-$CH_2$-COOH.

**55.** The composition of claim 47, wherein at least 50% of the R groups are -NH-CO-$CH_2$-$CH_2$-COOH.

**56.** The composition of claim 47, wherein at least 90% of the R groups are -NH-CO-$CH_2$-$CH_2$-COOH.

**57.** The composition of claim D2, wherein the capsular polysaccharide/adhesin has a molecular weight of greater than 25,000.

**58.** The composition of claim 47, wherein the capsular polysaccharide/adhesin has a molecular weight of greater than 100,000.

**59.** The composition of claim 47, wherein the capsular polysaccharide/adhesin is a homo-substituted polymer.

**60.** The composition of claim 47, wherein the capsular polysaccharide/adhesin is a hetero-substituted polymer.

**61.** The composition of claim 47, wherein R is selected from the group consisting of -NH-CO-$CH_2$-$CH_2$-$CH_2$-COOH, -NH-CO-$CH_2$-$CH_2$-$CH_2$-$CH_2$-COOH, and -NH-CO-$CH_2$-$CH_2$-$CH_2$-$CH_2$-$CH_2$-COOH.

**62.** The composition of claim 47, wherein R is selected from the group consisting of -NH-$CH_2$-$CH_2$-COOH, -NH-$CH_2$-$CH_2$-$CH_2$-COOH, -NH-$CH_2$-$CH_2$-$CH_2CH_2$-COOH, and -NH-$CH_2$-$CH_2$-$CH_2$-$CH_2$-$CH_2$-COOH.

**63.** The composition of claim 47, wherein R is selected from the group consisting of -NH-$CH_2$-$CH_2$-$(COOH)_2$, -NH-$CH_2$-$CH_2$-$CH_2$-$(COOH)_2$, -NH-$CH_2$-$CH_2$-$CH_2$-$CH_2$-$(COOH_2)$, and -NH-$CH_2$-$CH_2$-$CH_2$-$CH_2$-$CH_2$-$(COOH)_2$.

**64.** The composition of claim 47, wherein the capsular polysaccharide/adhesin is substantially free of teichoic acid.

**65.** The composition of claim 47, wherein the capsular polysaccharide/adhesin is formulated as a vaccine.

**66.** The composition of claim 47, further comprising a carrier compound conjugated to the capsular polysaccharide/adhesin.

**67.** The composition of claim 66, wherein the carrier compound is conjugated to the capsular polysaccharide/adhesin through a linker.

**68.** A composition comprising:

a capsular polysaccharide/adhesin having the following structure:

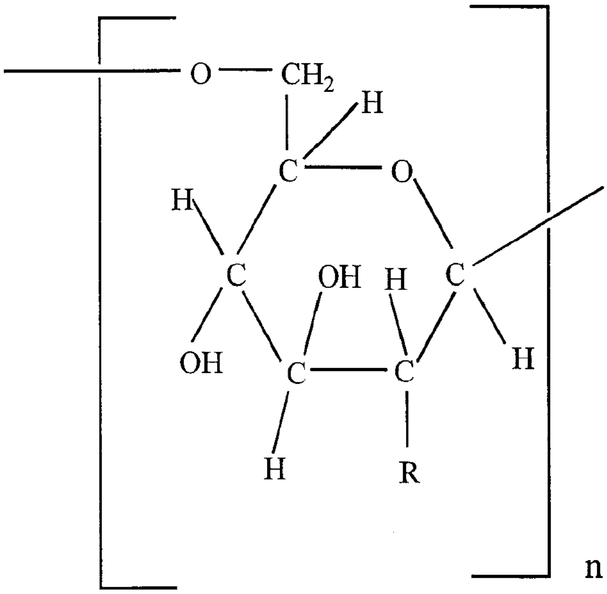

wherein n is an integer greater than or equal to 3, wherein R is selected from the group consisting of $-NH-CO-(CH_2)_m-COOH$, $-NH-(CH_2)_m-(COOH)_2$, $-NH-(CH_2)_m-COOH$, and $-NH_2$, wherein m is 2-5, and wherein at least 10% of the R groups are selected from the group consisting of $-NH-CO-(CH_2)_m-COOH$, $-NH-(CH_2)_m-(COOH)_2$, and $-NH-(CH_2)_m-COOH$ and wherein the capsular polysaccharide/adhesin is substantially free of teichoic acid.

**69.** The composition of claim 68, wherein at least 50% of the R groups are selected from the group consisting of $-NH-CO-(CH_2)_m-COOH$, $-NH-(CH_2)_m-(COOH)_2$, and $-NH-(CH_2)_m-COOH$.

**70.** The composition of claim 68, wherein at least 75% of the R groups are selected from the group consisting of $-NH-CO-(CH_2)_m-COOH$, $-NH-(CH_2)_m-(COOH)_2$, and $-NH-(CH_2)_m-COOH$.

**71.** The composition of claim 68, wherein at least 90% of the R groups are selected from the group consisting of $-NH-CO-(CH_2)_m-COOH$, $-NH-(CH_2)_m-(COOH)_2$, and $-NH-(CH_2)_m-COOH$.

**72.** The composition of claim 68, wherein at least 10% of the R groups are $-NH-CO-CH_2-CH_2-COOH$.

**73.** The composition of claim 68, wherein at least 50% of the R groups are $-NH-CO-CH_2-CH_2-COOH$.

**74.** The composition of claim 68, wherein at least 90% of the R groups are $-NH-CO-CH_2-CH_2-COOH$.

**75.** The composition of claim 68, wherein the capsular polysaccharide/adhesin has a molecular weight of greater than 25,000.

**76.** The composition of claim 68, wherein the capsular polysaccharide/adhesin has a molecular weight of greater than 30,000.

**77.** The composition of claim 68, wherein the capsular polysaccharide/adhesin has a molecular weight of greater than 100,000.

**78.** The composition of claim 68, wherein the capsular polysaccharide/adhesin is a homo-substituted polymer.

**79.** The composition of claim 68, wherein the capsular polysaccharide/adhesin is a hetero-substituted polymer.

**80.** The composition of claim 68, wherein R is selected from the group consisting of $-NH-CO-CH_2-CH_2-CH_2-COOH$, $-NH-CO-CH_2-CH_2-CH_2-CH_2-COOH$, and $-NH-CO-CH_2-CH_2-CH_2-CH_2-CH_2-COOH$.

**81.** The composition of claim 68, wherein R is selected from the group consisting of $-NH-CH_2-CH_2-COOH$, $-NH-CH_2-CH_2-CH_2-COOH$, $-NH-CH_2-CH_2-CH_2-CH_2-COOH$, and $-NH-CH_2-CH_2-CH_2-CH_2-CH_2-COOH$.

**82.** The composition of claim 68, wherein R is selected from the group consisting of $-NH-CH_2-CH_2-(COOH)_2$, $-NH-CH_2-CH_2-CH_2-(COOH_2)$, $-NH-CH_2-CH_2$ $-CH_2-CH_2-(COOH_2)$, and $-NH-CH_2-CH_2-CH_2-CH_2-CH_2-(COOH)_2$.

**83.** The composition of claim 68, wherein the capsular polysaccharide/adhesin is formulated as a vaccine.

**84.** The composition of claim 68, further comprising a carrier compound conjugated to the capsular polysaccharide/adhesin.

**85.** The composition of claim 84, wherein the carrier compound is conjugated to the capsular polysaccharide/adhesin through a linker.

**86.** A polysaccharide comprising:

a pure capsular polysaccharide/adhesin prepared according to the following method:
extracting a crude capsular polysaccharide/adhesin preparation from a bacterial culture,
isolating a high-molecular weight capsular polysaccharide/adhesin-enriched material from the crude capsular polysaccharide/adhesin preparation,
precipitating an impure capsular polysaccharide/adhesin from the high-molecular weight capsular polysaccharide/adhesin-enriched material,
incubating the impure capsular polysaccharide/adhesin with a base to produce a semi-pure capsular polysaccharide/adhesin preparation;
neutralizing the preparation with an acid, and
incubating the neutralized preparation in hydrofluoric acid to produce the pure impure capsular polysaccharide/adhesin.

**87.** The polysaccharide of claim 86, wherein the bacterial culture is a *Staphylococcus aureus* culture.

**88.** The polysaccharide of claim 86, wherein the bacterial culture is a coagulase negative *Staphylococci* culture.

**89.** A method of inducing active immunity to infection by *staphylococci* in a subject comprising:

administering to a subject an effective amount for inducing active immunity to *staphylococci* of the composition of any one of claims 1, 22, 34, 46, 68 and 86.

**90.** The method of claim 89, wherein the method is a method for inducing immunity to infection by *staphylococcus aureus.*

**91.** The method of claim 89, wherein the method is a method for inducing immunity to infection by *staphylococcus epidermis.*

**92.** A method of inducing passive immunity to infection by *staphylococcus aureus* in a subject comprising:

administering to a subject an effective amount for inducing opsonization of *staphylococcus aureus* of an anti capsular polysaccharide/adhesin antibody.

**93.** A method of inducing active immunity to infection by *staphylococcus aureus* in a subject comprising:

administering to a subject an effective amount for inducing active immunity to *staphylococcus aureus* of a high molecular weight polysaccharide having a polyglucosamine backbone wherein at least 10% of individual glucosamine units of the polyglucosamine backbone are conjugated to succinate.

**94.** The method of claim 93, wherein the polyglucosamine backbone is in a $\beta$-1-6 formation.

**95.** A method of preparing a pure polysaccharide comprising:

extracting a crude capsular polysaccharide/adhesin preparation from a bacterial culture,
isolating a high-molecular weight capsular polysaccharide/adhesin-enriched material from the crude capsular polysaccharide/adhesin preparation,
precipitating an impure capsular polysaccharide/adhesin from the high-molecular weight capsular polysaccharide/adhesin-enriched material,
incubating the impure capsular polysaccharide/adhesin with a base or acid to produce a semi-pure capsular polysaccharide/adhesin preparation;
neutralizing the preparation, and
treating the neutralized preparation to produce the pure capsular polysaccharide/adhesin.

**96.** A composition, comprising:

an anti-capsular polysaccharide/adhesin$_{pure}$ antibody, wherein the antibody induces opsonization of *staphylococcus aureus* and wherein the antibody does not crossreact with an impure PS/A preparation.

**97.** The composition of claim 96, wherein the anti-capsular polysaccharide/adhesin$_{pure}$ antibody is a monoclonal antibody.

**98.** The composition of claim 96, wherein the anti-capsular polysaccharide/adhesin$_{pure}$ antibody is a polyclonal antibody.

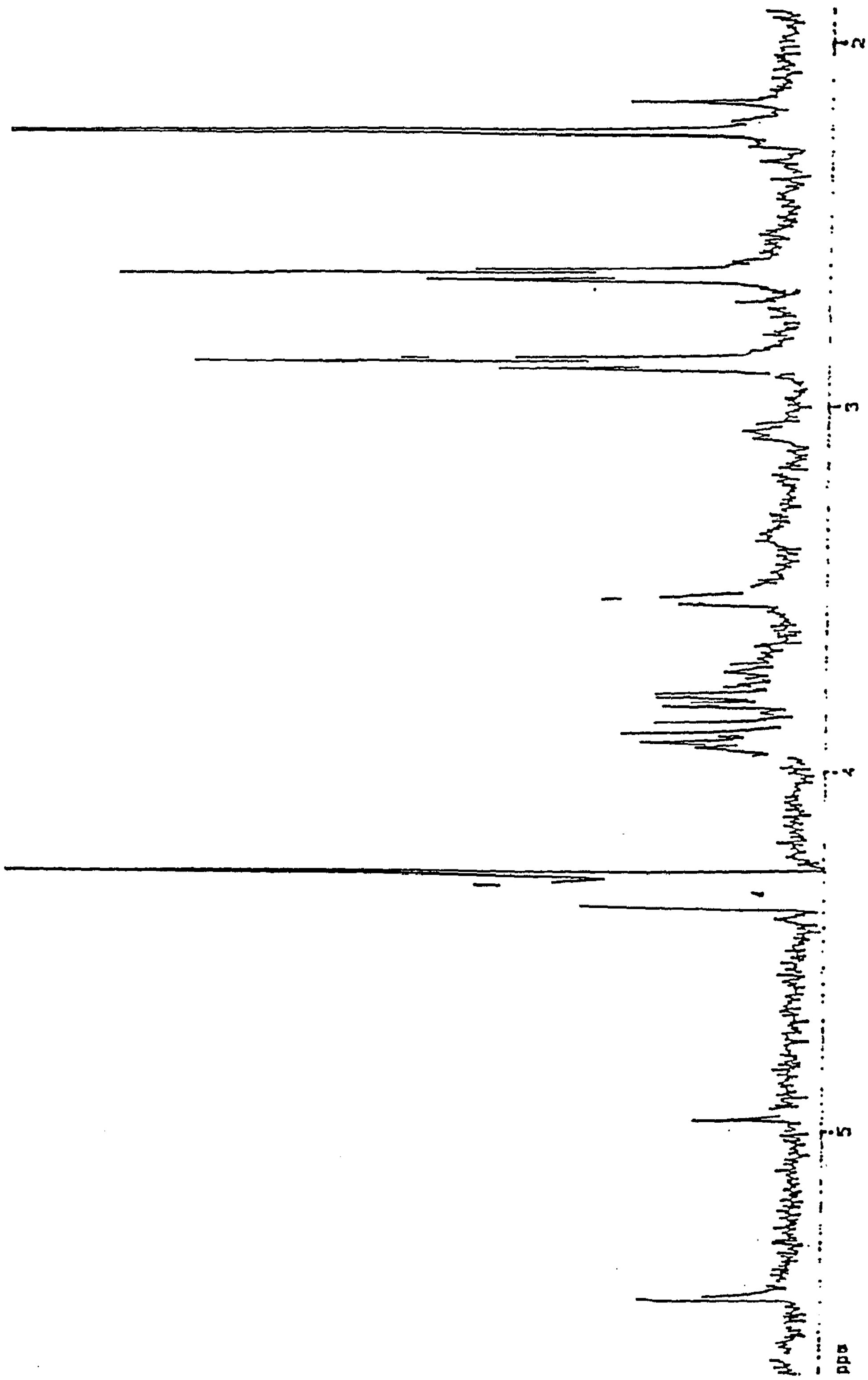


Figure 1

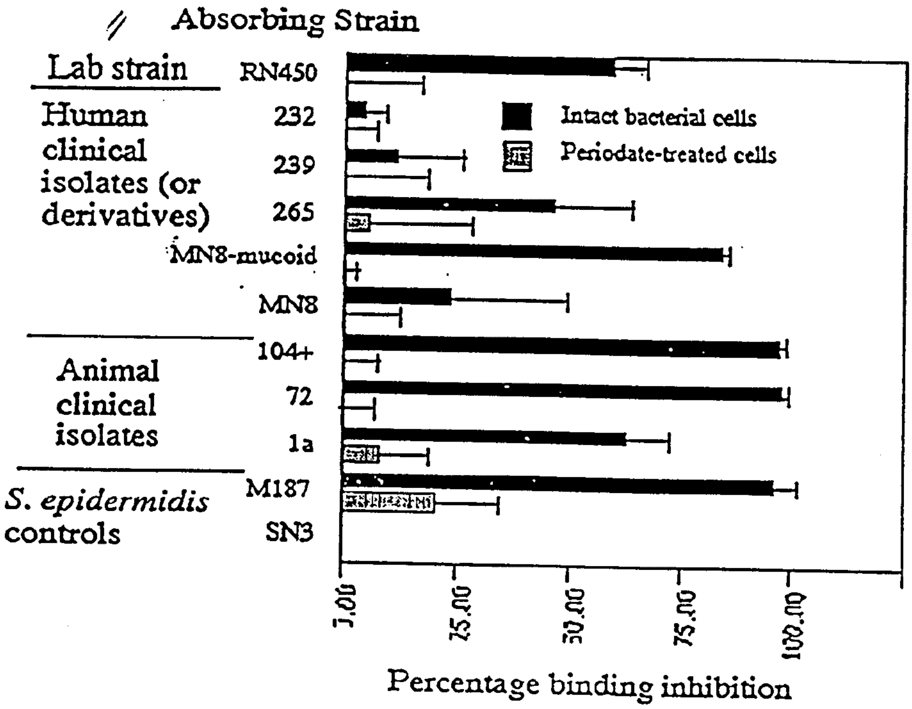
Absorbing Strain
Lab strain
RN450
Human clinical isolates (or derivatives)
232
239
265
MN8-mucoid
MN8
Animal clinical isolates
104+
72
1a
S. epidermidis controls
M187
SN3
Intact bacterial cells
Periodate-treated cells
Percentage binding inhibition

Figure 2

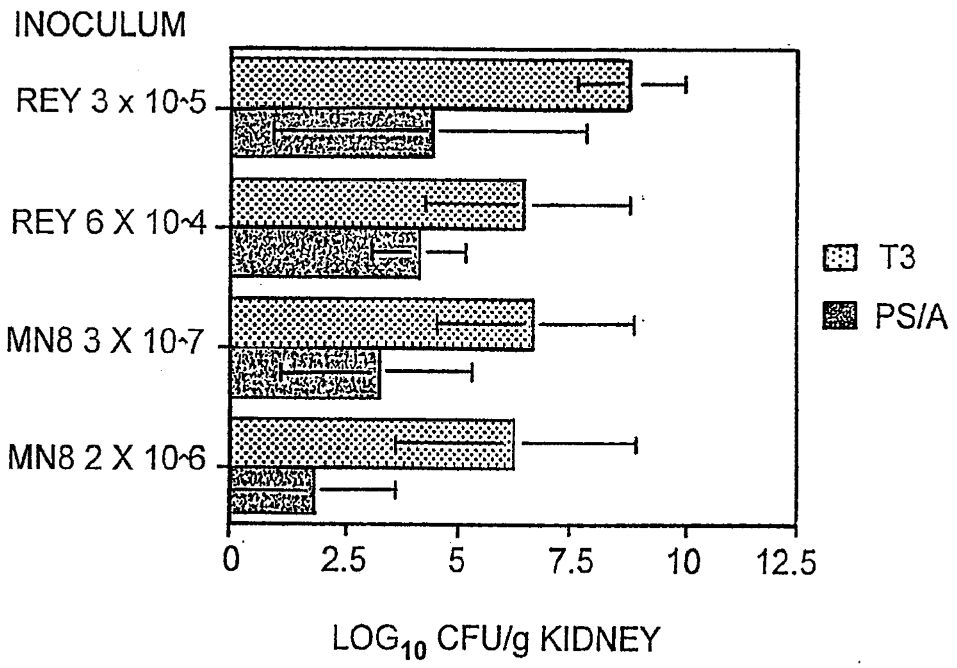


ACTIVE IMMUNITY: SIGNIFICANT DIFFERENCE NOTED BETWEEN PS/A AND T3 IMMUNIZED SWISS WEBSTER MICE (n=5, $p \leq 0.037$).

*Fig. 3*

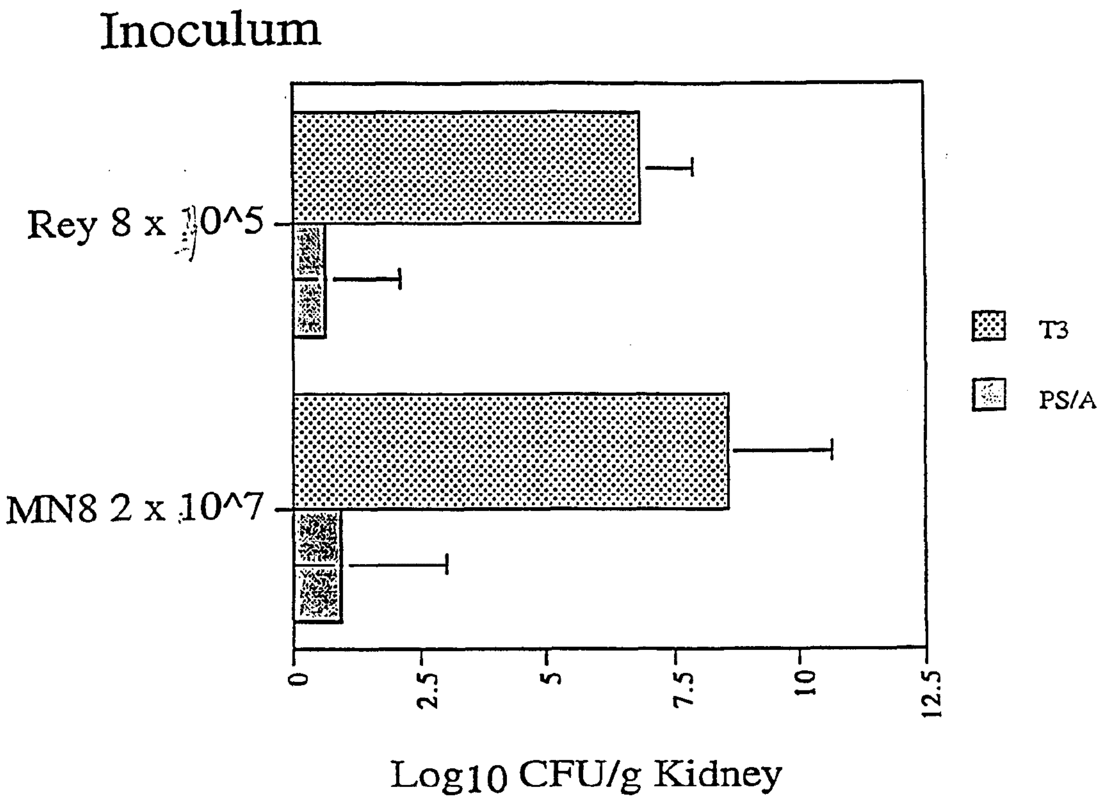


Passive Immunity: Significant differences noted between anti-PS/A and anti-T3 immunized Swiss Webster Mice (n=5, $p \leq 0.0002$).

Figure 4

Gel#

Date: | Descript.:

| | |
|---|---|
| V/A: | |
| Agar%: | 0.7 |
| Time: | |
| Temp.: | RT |

1. MARKER 1kb LADDER
2. NO DNA 20μl
3. *ica*A-C
4. RP62A
5. MN8
6. MN8-MUC.
7. RN450
8. REYNOLDS
9. BECKER
10. 1A
11.
12.
13.
14.
15.
16.
17.
18.
19.
20.
21.
22.
23.
24.
25.
26.
27.
28.
29.
30.
31.
32.

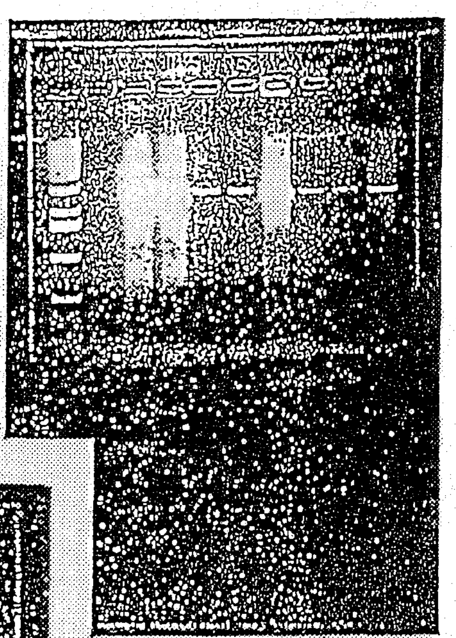

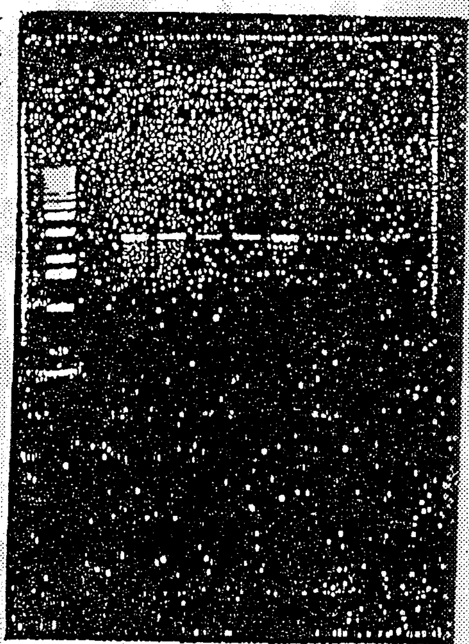

RELOADED GEL WITH REDUCING CORE OF PCR PRODUCT FROM *ica*A-C, RP62A AND RN450.

*Fig. 5*

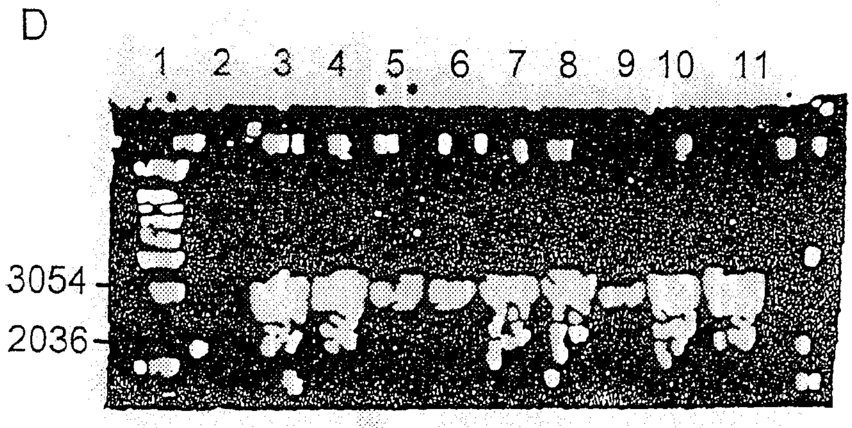


*Fig. 6*

European Patent Office

# PARTIAL EUROPEAN SEARCH REPORT

which under Rule 63 of the European Patent Convention shall be considered, for the purposes of subsequent proceedings, as the European search report

Application Number
EP 08 10 1785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MACK D ET AL: "The intercellular adhesin involved in biofilm accumulation of Staphylococcus epidermidis is a linear beta-1,6-linked glucosaminoglycan: Purification and structural analysis" JOURNAL OF BACTERIOLOGY, vol. 178, no. 1, January 1996 (1996-01), pages 175-183, XP000867576 * the whole document * ----- | 1,8,11, 19-22, 26,29, 34,38, 41,68, 75,78 | INV. A61K39/085 C07H5/06 C07H11/00 C07H13/12 C08B37/00 |
| X | US 4 902 616 A (BOUVET ANNE ET AL) 20 February 1990 (1990-02-20) * the whole document * ----- | 92 | |
| X | WESSELS M R ET AL: "Isolation and characterization of type IV group B Streptococcus capsular polysaccharide." INFECTION AND IMMUNITY, (1989 APR) 57 (4) 1089-94., 1989, XP002129163 * page 1090, column 1, line 31 - line 56 * ----- -/-- | 95 | |

TECHNICAL FIELDS SEARCHED (IPC)

A61K
C07H
C08B

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| Munich | 14 July 2008 | Wagner, René |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 08 10 1785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEE J C ET AL: "Chemical characterization and immunogenicity of capsular polysaccharide isolated from mucoid Staphylococcus aureus." INFECTION AND IMMUNITY, (1987 SEP) 55 (9) 2191-7., 1987, XP002129164 the whole document, especially discussion | 96-98 | |
| D,A | WO 90/03398 A (BRIGHAM & WOMENS HOSPITAL) 5 April 1990 (1990-04-05) * page 1, line 1 - line 9 * * page 5, line 30 - page 7, line 5 * * page 13, line 15 - page 14, line 14 * * page 17, line 20 - page 18, line 22 * * page 23, line 4 - line 10 * * claims 1,2,13-17,24-30 * | 1-98 | TECHNICAL FIELDS SEARCHED (IPC) |
| A,D | KOJIMA Y ET AL: "Antibody to the capsular polysaccharide/adhesin protects rabbits against catheter-related bacteremia due to coagulase-negative staphylococci" J INFECT DIS, vol. 162, no. 2, August 1990 (1990-08), pages 435-41, XP000867597 * the whole document * | 1-98 | |
| A | FATTOM A ET AL: "Effect of conjugation methodology, carrier protein, and adjuvants on the immune response to Staphylococcus aureus capsular polysaccharides" VACCINE,GB,BUTTERWORTH SCIENTIFIC. GUILDFORD, vol. 13, no. 14, 1 October 1995 (1995-10-01), pages 1288-1293, XP004057429 ISSN: 0264-410X * the whole document * | 17,18, 32,33, 44,45, 66,67, 84,85 | |

-/--

1

EPO FORM 1503 03.82 (P04C10)

European Patent Office

# PARTIAL EUROPEAN SEARCH REPORT

Application Number
EP 08 10 1785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FR 2 640 628 A (COMMISSARIAT ENERGIE ATOMIQUE ;CENTRE NAT RECH SCIENT (FR)) 22 June 1990 (1990-06-22)<br>* the whole document *<br>----- | 1-15, 22-30, 34-42, 46-63, 68-82 | |
| P,X | MCKENNEY D ET AL: "Broadly protective vaccine for Staphylococcus aureus based on an in vivo-expressed antigen."<br>SCIENCE,, May 1999 (1999-05), XP002129165<br>* the whole document *<br>----- | 1-98 | |
| P,X | MCKENNEY D ET AL: "The ica locus of Staphylococcus epidermidis encodes production of the capsular polysaccharide /adhesin."<br>INFECTION AND IMMUNITY, (1998 OCT) 66 (10) 4711-20., 1998, XP002129166<br>* the whole document *<br>----- | 1-98 | TECHNICAL FIELDS SEARCHED (IPC) |
| A | GEHRON M J ET AL: "DETERMINATION OF THE GRAM POSITIVE BACTERIAL CONTENT OF SOILS AND SEDIMENTS BY ANALYSIS OF TEICHOIC-ACID COMPONENTS"<br>JOURNAL OF MICROBIOLOGICAL METHODS, vol. 2, no. 3, 1984, pages 165-176, XP002487992<br>ISSN: 0167-7012<br>* the whole document *<br>----- | 1-98 | |
| A | WICKEN A J ET AL: "CHARACTERIZATION OF GROUP N STREPTOCOCCUS LIPO TEICHOIC-ACID"<br>INFECTION AND IMMUNITY, vol. 11, no. 5, 1975, pages 973-981, XP002487993<br>ISSN: 0019-9567<br>* the whole document *<br>----- | 1-98 | |

1

EPO FORM 1503 03.82 (P04C10)

European Patent Office

INCOMPLETE SEARCH SHEET C

Application Number
EP 08 10 1785

Although claims 89-94 are directed to a method of treatment of the human/animal body, the search has been carried out and based on the alleged effects of the compound/composition.

-----

## ANNEX TO THE EUROPEAN SEARCH REPORT ON EUROPEAN PATENT APPLICATION NO. EP 08 10 1785

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2008

| Patent document cited in search report | | Publication date | Patent family member(s) | | | Publication date |
|---|---|---|---|---|---|---|
| US 4902616 | A | 20-02-1990 | CA | 1320466 | C | 20-07-1993 |
| | | | DE | 3875850 | D1 | 17-12-1992 |
| | | | DE | 3875850 | T2 | 09-06-1993 |
| | | | DK | 433488 | A | 04-02-1989 |
| | | | EP | 0302781 | A1 | 08-02-1989 |
| | | | ES | 2052759 | T3 | 16-07-1994 |
| | | | FR | 2619122 | A1 | 10-02-1989 |
| | | | GR | 3006613 | T3 | 30-06-1993 |
| | | | JP | 1128793 | A | 22-05-1989 |
| | | | PT | 88166 | A | 30-06-1989 |
| WO 9003398 | A | 05-04-1990 | AU | 4343089 | A | 18-04-1990 |
| | | | CA | 1317288 | C | 04-05-1993 |
| | | | EP | 0436648 | A1 | 17-07-1991 |
| | | | JP | 4501718 | T | 26-03-1992 |
| | | | US | 5055455 | A | 08-10-1991 |
| FR 2640628 | A | 22-06-1990 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 5055455 A, Gerald B. Pier
- US 4356170 A
- US 4663160 A, Tsay
- US 4619828 A, Gordon
- US 4808700 A, Anderson and Clements
- US 4711779 A, Porro and Costantino
- US 5567610 A, Borrebaeck
- US 565354 A, Ostberg
- US 5571893 A, Baker
- US 5569825 A, Lonberg
- EP 0239400 A
- US 4946778 A, Ladner
- US 4462334 A, Moore
- US 4355023 A, Ehrilch
- US 4470925 A
- US 4452775 A, Kent
- US 4667014 A, Nestor
- US 4748034 A
- US 5239660 A, Leonard
- US 3832253 A, Higuchi
- US 3854480 A, Zaffaroni
- US 4737456 A
- US 3940475 A
- US 3645090 A

### Non-patent literature cited in the description

- **KOJIMA, Y. ; M. TOJO ; D. A. GOLDMANN ; T. D. TOSTESON ; G. B. PIER.** *J Infect Dis.,* 1990, vol. 162, 435
- **TOJO, M. ; N. YAMASHITA ; D. A. GOLDMANN ; G. B. PIER.** *J Infect Dis.,* 1988, vol. 157, 713
- **GOLDMANN, D. A. ; G. B. PIER.** *Clin Microbiol Rev.,* 1993, vol. 6, 176
- **KELETI, G. ; W.H. LEDERER.** Handbook of Micromethods for the Biological Sciences. Van Nostrand Reinhold Co, 1974
- **LOWRY, O.H. ; N.R. ROBERTS ; K.Y. LEINER ; M.L. WU ; A. L. FARR.** *Biol. Chem.,* 1954, vol. 207, 1
- **SZU, S.C. ; X. LI ; A.L. STONE ; J.B. ROBBINS.** Relation between structure and immunologic properties of the Vi capsular polysaccharide. *Infection and Immunity,* 1991, vol. 59, 4555-4561
- **DIFABIO, J. L ; MICHON, F. ; BRISSON, J.R. ; JENNINGS, H.J. ; WESSELS, M.R. ; BENEDI, V.J. ; KASPER, D.L.** Structure of the capsular polysaccharide antigen of type IV groups B Streptococcus. *Canadian Journal of Chemistry,* 1989, vol. 67, 877-882
- **LUNDQUIST et al.** *J. Carbohydrate Chem.,* 1991, vol. 10, 377
- **KOCHETKOW.** *Carbohydrate Research,* 1986, vol. 146, C1
- **ISEBELL et al.** Methods of Carbohydrate Chemistry. Academic Press, 1962
- Immunoglobulin-producing hybrid cell lines. **OI VT.** selected methods in cellular biology. Freeman
- **KOZBER.** *J. Immunol.,* 1984, vol. 133, 3001
- **BRODEUR et al.** Monoclonal Antibody Production Techniques and Applications. Marcel Dekker, Inc, 1987, 51-63
- **BOERNER.** *J. Immunol.,* 1991, vol. 147, 86-95
- **JAKOBOVITS et al.** *PNAS USA,* 1993, vol. 90, 2551
- **JAKOBOVITS et al.** *Nature,* 1993, vol. 362, 255-258
- **BRUGGERMANN et al.** *Year in Immuno.,* 1993, vol. 7, 33
- **L. RIECHMANN et al.** *Nature,* 1988, vol. 332, 323
- **M. S. NEUBERGER et al.** *Nature,* 1985, vol. 314, 268
- **CLARK, W.R.** The Experimental Foundations of Modern Immunology. Wiley & Sons, Inc, 1986
- **ROITT, I.** Essential Immunology. Blackwell Scientific Publications, 1991
- **KLEIN.** Immunology. John Wiley, 1982
- **CLARK, W.R.** The Experimental Foundations of Modern Immunology. Wiley & Sons, Inc, 1986
- **ROITT, 1.** Essential Immunology. Blackwell Scientific Publications, 1991
- **WAHL et al.** *J. Nucl. Med.,* 1983, vol. 24, 316-325
- **WARD et al.** *Nature,* 1989, vol. 341, 644-646
- **TIJSSEN.** Practice and Theory of Enzyme Immunoassays. Elsevieer, 1985
- **HOCHMAN et al.** *Biochemistry,* 1973, vol. 12, 1130
- **SHARON et al.** *Biochemistry,* 1976, vol. 15, 1591

- **HART et al.** *J. Biol. Chem.,* 1994, vol. 269, 12468
- **HEILMANN, C. ; O. SCHWEITZER ; C. GERKE ; N. VANITTANAKOM ; D. MACK ; F. GOTZ.** Molecular basis of intercellular adhesion in the biofilm-forming Staphylococcus epidermidis. *Molec. Microbiol.,* 1996, vol. 20, 1083
- **GOODMAN ; GILMAN'S.** Pharmacological Basics of Therapeutics. McGraw Hill Inc, 1993
- *European Journal of Biochemistry,* 1981, vol. 116, 359-364
- **FOURNIER, J.-M. ; K. HANNON ; M. MOREAU ; W.W. KARAKAWA ; W.F. VANN.** Isolation of type 5 capsular polysaccharide from Staphylococcus aureus. *Ann. Inst. Pasteur/Microbiol. (Paris,* 1987, vol. 138, 561-567
- **RHIZOBIUM MELILOTI.** *Cell,* 1989, vol. 65, 661-672
- **WESSELS, M.R. ; V. POZSGAY ; D.L. KASPER ; H. J. JENNINGS.** Structure and immunochemistry of an oligosaccharide repeating unit of the capsular polysaccharide of type III group B Streptococcus. *Journal of Biological Chemistry,* 1987, vol. 262, 8262-8267
- **KNIREL, Y.A. ; N.A. PARAMONOV ; E.V. VINOGRADOV ; A.S. SHASHKOW ; B.A. N.K. KOCHETKOV ; E.S. STANISLAVSKY ; E.V. KHOLODKOVA.** Somatic antigens of Pseudomonas aeruginosa The structure of O-specific polysaccharide chains of lipopolysaccharides of P. aeruginosa 03(Lanyi), 025 (Wokatsch) and Fisher immunotypes 3 and 7. *European Journal of Biochemistry,* 1987, vol. 167, 549
- **KENNE, L. ; B. LINDBERG ; K. PETERSSON.** Structural studies of the O-specific side-chains of the Shigella sonnei phase I lipopolysaccharide. *Carbohydrate Research,* 1980, vol. 78, 119-126
- **LINDBERG, B. ; LINDQVIST, B. ; LONNGREN, J. ; POWELL, D.A.** Structural studies of the capsular polysaccharide from Streptococcus pneumoniae type 1. *Carbohydrate Research,* 1980, vol. 78, 111-117
- **JENNINGS, H.J. ; C. LUGOWSKI ; N. M. YOUNG.** Structure of the complex polysaccharide C-substance from Streptococcus pneumoniae type 1. *Biochemistry,* 1980, vol. 19, 4712-4719
- **KRIEGLER, M.** Gene Transfer and Expression, A Laboratory Manual. W.H. Freeman Co, 1990
- Methods in Molecular Biology. Humana Press, Inc, 1991, vol. 7
- **FRALEY et al.** *Trends Biochem. Sci.,* 1981, vol. 6, 77
- **GREGORIADIS, G.** *Trends in Biotechnology,* 1985, vol. 3, 235-241
- **HUSSAIN, M. ; J. G. M. HASTINGS ; P. J. WHITE.** A chemically defined medium for slime production by coagulase-negative Staphylococci. *J. Med Microbiol.,* 1991, vol. 34, 143
- **HUSSAIN, M. ; J. G. M. HASTINGS ; P. J. WHITE.** A chemically defined medium for slime production by coagulase-negative Staphylococci. *J. Med. Microbiol.,* 1991, vol. 34, 143
- **MULLER, E. ; J. HUEBNER ; N. GUTIERREZ ; S. TAKEDA ; D. A. GOLDMANN ; G. B. PIER.** Isolation and characterization of transposon mutants of Staphylococcus epidermidis deficient in capsular polysaccharide/adhesin and slime. *Infect Immun.,* 1993, vol. 61, 551
- **TOJO, M. ; N. YAMASHITA ; D. A. GOLDMANN ; G. B. PIER.** Isolation and characterization of a capsular polysaccharide/adhesin from Staphylococcus epidermidis. *J Infect Dis.,* 1988, vol. 157, 713
- **KELETI, G. ; W.H. LEDERER.** Handbook of Micromethods for the Biological Sciences. Van Nostrand Reinhold Co, 1974
- **KOJIMA, Y. ; M. TOJO ; D. A. GOLDMANN ; T. D. TOSTESON ; G. B. PIER.** Antibody to the capsular polysaccharide/adhesin protects rabbits against catheter related bacteremia due to coagulase-negative staphylococci. *J Infect Dis.,* 1988, vol. 162, 435
- **TOJO, M. ; N. YAMASHITA ; D. A. GOLDMANN ; G. B. PIER.** Isolation and characterization of a capsular polysaccharide/adhesin from Staphylococcus epidermidis. *J Infect Dis.,* vol. 157, 713
- **TAKEDA, S. ; G. B. PIER ; Y. KOJIMA ; M. TOJO ; E. MULLER ; T. TOSTESON ; D. A. GOLDMANN.** Protection against endocarditis due to Staphylococcus epidermidis by immunization with capsular polysaccharide/adhesin. *Circulation,* 1991, vol. 84, 2539
- **HEILMANN, C. ; O. SCHWEITZER ; C. GERKE ; N. VANITTANAKOM ; D. MACK ; F. GOTZ.** Molecular basis of intercellular adhesion in the biofilm-forming Staphylococcus epidermidis. *Molec. Microbiol.,* 1996, vol. 20, 1083